# EUROPEAN PATENT APPLICATION

(11) **EP 2 762 124 A1**
(43) Date of publication of application: **06.08.2014**
(21) Application number: 14152562.6
(22) Date of filing: 24.01.2014
(51) Int. Cl.: A61J 1/03, A61K 9/20, C07D 333/40, A61K 31/381

(54) **Packaging comprising administration units of polymorphs, amorphous forms or solvates**

(30) Priority: 31.01.2013 EP 13153505; 31.01.2013 EP 13153517
(71) Applicant: IP Gesellschaft für Management mbH, 50931 Köln (DE)
(72) Inventor: Trinius, Frank, 50931 Köln (DE)

(57) **Abstract**

The invention relates to an administration unit comprising (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • methanol, Compound (1) • ethanol • isopropanol, Compound (1) • acetone, Compound (1) • ethylacetate, Compound (1) • isopropylacetate, Compound (1) • ethylacetate • 2-methyl THF, Compound (1) • ethanol, Compound (1) • n-butylacetate, Compound (1) • heptane, Compound (1) • 2-butanone, or Compound (1) • methylacetate and to a packaging comprising the administration unit according to the invention.

## Description

### CROSSREFERENCES

The present application claims priorities of European patent application no. EP13153505 filed on January 31, 2013; and European patent application no. EP13153517 filed on January 31, 2013.

### BACKGROUND ART

Many pharmaceuticals are marketed without any packaging. In various countries, small drug shops are a common source of medicine where stocking of prescription-only medicines and unpackaged tablets is the norm (Goodman C et al., Health Policy Plan. 2007 Nov;22(6):393-403). Furthermore, self-medication is common practice in various countries. For example, the most frequently proposed drugs to treat male urethritis are: ampicillin 250-mg capsules (44%); oxytetracyline 250-mg capsules (24%); and cotrimoxazole 450-mg tablets (12%), and these drugs are frequently sold unpackaged (Sow PSet al., Int J Infect Dis. 2002 Jun;6(2):108-12).

In Germany and other European countries, numerous pharmaceuticals are marketed in packaging wherein every single administration unit is individually packaged in a single packaging. Examples include but are not limited to Frubiase^{®} Calcium (Boehringer Ingelheim) marketed as a liquid administration unit that is individually packaged in a glass ampoule; Orthomol Immun^{®} Direktgranulat (Orthomol) marketed as administration unit in granulate form that is individually packaged in a bag; Orthomol Vital m^{®} (Orthomol) marketed as a liquid administration unit that is individually packaged in a bottle having a lid containing an individually packaged tablet; Vitasprint^{®} (Pfizer) and Celestamine^{®} (MSD Sharp & Dohme) each marketed as a liquid administration unit that is individually packaged in a bottle; Alka-Seltzer^{®} (Bayer) marketed as effervescent tablet that is individually packaged in a bag; Aspirin^{®} Complex (Bayer), Aspirin^{®} Effect (Bayer), Helmex^{®} (Infectopharm), Monuril^{®} Granulat (Pierre Fabre Pharma) each marketed as administration unit in granulate form that individually packaged in a bag; ellaOne^{®} (HRA Pharma), Levonelle^{®} (Bayer Schering Pharma); Pidana^{®} (HRA Pharma), Fungata^{®} (Pfizer), and Canesten^{®} Gyn once (Bayer) each marketed as a single tablet that is individually packaged in a blister. Furthermore, numerous single-to-use syringes are marketed individually packaged.

It is an object of the invention to provide administration units containing drugs that have advantages compared to conventional administration units and conventional drugs, respectively. It is also an object of the invention to provide packaging containing administration units containing drugs that have advantages compared to conventional packaging. These objects have been achieved by the present invention.

### SUMMARY OF THE INVENTION

The present invention relates to different forms of a drug (active pharmaceutical ingredient), such as polymorphs and/or solvates thereof. The present invention covers various aspects that are numbered (i) and (ii). Aspects (i) and (ii) are separate from one another.

The present invention relates to
⊙ an administration unit comprising (i) polymorphic Form M of Compound (1), polymorphic Form H of Compound (1),
polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate;
⊙ a packaging comprising one or more of such administration units;
⊙ a method for manufacturing such an administration unit;
⊙ a method for manufacturing such a packaging; and
⊙ particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.

### GENERAL DEFINITIONS

To avoid unnecessary repetitions, the description refers to the following abbreviations the meaning of which is defined hereinafter: The present invention generally relates to the following compound, in the following abbreviated as 'Compound (1)':

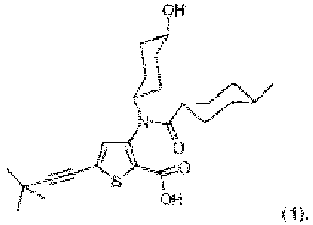

WO 2013/016490 discloses polymorph forms M, H, P, X and ZA of Compound (1). WO 2013/016491 discloses various solvates of Compound (1). WO 2013/016492 discloses co-crystals of Compound (1). WO 2013/016499 discloses a method of preparing Compound (1). WO 2013/016501 discloses a pharmaceutical composition of polymorphic form M or tromethamine salt of Compound (1) and a filler. The disclosure of WO 2013/016490, WO 2013/016491, WO 2013/016492, WO 2013/016499, and WO 2013/016501 is incorporated herein by reference.

Aspect (i) of the invention relates to amorphous and polymorphic forms of Compound (1), namely to amorphous Form of Compound (1); and polymorphic forms selected from the group consisting of polymorphic Form M of Compound (1); polymorphic Form H of Compound (1); polymorphic Form P of Compound (1); polymorphic Form X of Compound (1); and polymorphic Form ZA of Compound (1). Amorphous Form, polymorphic Form M, polymorphic Form H, polymorphic Form P, polymorphic Form X, and polymorphic Form ZA are specific polymorphic forms falling within the definition of Compound (1). Aspect (i) of the invention is separate from aspect (ii) of the invention.

Amorphous Form of Compound (1) (= 'amorphous Form') is preferably characterized as having a solid state C¹³ nuclear magnetic spectroscopy (NMR) spectrum
- having peaks at 161.1, 132.9, 106.5, 43.3, 31.2, and 23.3; and/or
- substantially the same as that shown in FIG. 9 of WO2013016490.

Polymorphic Form M of Compound (1) (='polymorphic Form M') is preferably characterized as having
(a) an X-ray powder diffraction pattern (obtained at room temperature using Cu K alpha radiation)
   - with the most intense characteristic peak expressed in 2-theta ± 0.2 at 19.6; and/or
   - with characteristic peaks expressed in 2-theta ± 0.2 at the following positions: 19.6, 16.6, 18.1, 9.0, 22.2, and 11.4; and/or
   - with characteristic peaks expressed in 2-theta ± 0.2 at the following positions with relative intensities in parentheses: 19.6 (100.0%), 16.6 (72.4%), 18.1 (59.8%), 9.0 (47.6%), 22.2 (39.9%), and 11.4 (36.6%); and/or
   - substantially the same as that shown in FIG. 2 of WO2013016490; and/or
(b) an endothermic peak in differential scanning calorimetry (DSC) at 230 ± 2 °C; and/or
(c) a solid state C¹³ nuclear magnetic spectroscopy (NMR) spectrum
   - having peaks in at 177.3, 134.3, 107.4, 56.5, 30.7, and 25.3; and/or
   - substantially the same as that shown in FIG. 6 of WO2013016490.

Polymorphic Form H of Compound (1) (='polymorphic Form H') is preferably characterized as having
(a) an X-ray powder diffraction pattern (obtained at room temperature using Cu K alpha radiation)
   - with characteristic peaks expressed in 2-theta ± 0.2 at 6.6 and 17.3, wherein the peak at 6.6 is the most intense peak; and/or
   - with characteristic peaks expressed in 2-theta ± 0.2 at the following positions: 6.6, 18.7, 8.5, 17.3, 15.8, and 19.4; and/or
   - with characteristic peaks expressed in 2-theta ± 0.2 at the following positions with relative intensities in parentheses: 6.6 (100.0%), 18.7 (87.8%), 8.5 (66.7%), 17.3 (58.4%), 15.8 (39.9%), and 19.4 (29.8%); and/or
   - substantially the same as that shown in FIG. 3 of WO2013016490; and/or
(b) an endothermic peak in differential scanning calorimetry (DSC) at 238 ± 2 °C; and/or
(c) a solid state C¹³ nuclear magnetic spectroscopy (NMR) spectrum
   - having peaks at 162.2, 135.9, 131.1, 109.5, 45.3, and 23.9; and/or
   - substantially the same as that shown in FIG. 7 of WO2013016490.

Polymorphic Form P of Compound (1) (='polymorphic Form P') is preferably characterized as having
(a) an X-ray powder diffraction pattern (obtained at room temperature using Cu K alpha radiation)
   - with characteristic peaks expressed in 2-theta ± 0.2 at 7.0 and 15.8, wherein the peak at 7.0 is the most intense peak; and/or
   - with characteristic peaks expressed in 2-theta ± 0.2 at the following positions: 7.0, 15.8, 9.8, 19.3, 8.5, and 21.9; and/or
   - with characteristic peaks expressed in 2-theta ± 0.2 at the following positions with relative intensities in parentheses: 7.0 (100%), 15.8 (21.9%), 9.8 (14.6%), 19.3 (11.9%), 8.5 (10.5%), and 21.9 (9.5%); and/or
   - substantially the same as that shown in FIG. 4 of WO2013016490; and/or
(b) an endothermic peak in differential scanning calorimetry (DSC) at 160 ± 2 °C; and/or
(c) a solid state C¹³ nuclear magnetic spectroscopy (NMR) spectrum
   - having peaks at 161.5, 133.6, 105.8,44.4,31.1 and 22.1; and/or
   - substantially the same as that shown in FIG. 8 of WO2013016490.

Polymorphic Form X of Compound (1) (='polymorphic Form X') is preferably characterized as having
(a) an X-ray powder diffraction pattern (obtained at room temperature using Cu K alpha radiation)
   - with characteristic peaks expressed in 2-theta ± 0.2 at 7.5 and 12.1; and/or
   - with characteristic peaks expressed in 2-theta ± 0.2 at the following positions: 7.5, 12.1, 13.0, 13.8, 16.2, and 19.7; and/or.
   - substantially the same as that shown in FIG. 9 of WO2013016490.

Polymorphic Form ZA of Compound (1) (='polymorphic Form ZA') is preferably characterized as having
(a) an X-ray powder diffraction pattern (obtained at room temperature using Cu K alpha radiation)
   - with characteristic peaks expressed in 2-theta ± 0.2 at 5.2 and 10.2; and/or
   - with characteristic peaks expressed in 2-theta ± 0.2 at the following positions: 5.2, 10.2, 16.5, 18.6, 19.8, and 20.3; and/or
   - substantially the same as that shown in FIG. 10 of WO2013016490.

Aspect (ii) of the invention relates to solvates of Compound (1), namely to Compound (1) • H₂O, wherein the Compound (1) • H₂O are preferably in a molar ratio of 1:0.5 to 1:3 (Compound (1) • H₂O), particularly Compound (1) • hydrate A or Compound (1) • hydrate B; Compound (1) • methanol; Compound (1) • ethanol • isopropanol; Compound (1) • acetone; Compound (1) • ethylacetate, particularly Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G; Compound (1) • isopropylacetate; Compound (1) • ethylacetate • 2-methyl THF, particularly Compound (1) • ethylacetate • 2-methyl THF (ethylacetate/2-methyl THF:70%/30% w/w) or Compound (1) • ethylacetate • 2-methyl THF (ethylacetate/2-methyl THF: 90%/10% w/w); Compound (1) • ethanol; Compound (1) • n-butylacetate, particularly Compound (1) • n-butylacetate Solvate A, Compound (1) • n-butylacetate Solvate B, Compound (1) • n-butylacetate Solvate C; Compound (1) • heptane, particularly Compound (1) • heptane Solvate A, Compound (1) • heptane Solvate B, Compound (1) • heptane Solvate C, Compound (1) • heptane Solvate D, Compound (1) • heptane Solvate E; Compound (1) • 2-butanone; and Compound (1) • methylacetate. Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, and Compound (1) • methylacetate are specific forms falling within the definition of Compound (1). Aspect (ii) of the invention is separate from aspect (i) of the invention.

Hydrates of Compound (1) (='Compound (1) • H₂O') are preferably characterized as having
(a) "hydrate A": an X-ray powder diffraction pattern (obtained at room temperature using Cu K alpha radiation)
   - with characteristic peaks expressed in 2-theta ± 0.2 at the following positions: 20.3 and 9.1; and/or
   - with characteristic peaks expressed in 2-theta ± 0.2 at the following positions: 20.3, 9.1, 18.4, 7.6, 19.6, and 14.6; and/or
   - with characteristic peaks expressed in 2-theta ± 0.2 at the following positions with relative intensities in parenthesis: 20.3 (100.0%), 9.1 (41.1%), 18.4 (31.5%) 7.6 (27.3%), 19.6 (25.7%), and 14.6 (24.4%); and/or
   - substantially the same as that shown in FIG. 1 of WO2013016491; and/or
(b) "hydrate B": an X-ray powder diffraction pattern (obtained at room temperature using Cu K alpha radiation)
   - with characteristic peaks expressed in 2-theta ± 0.2 at the following positions: 19.0 and 9.9; and/or
   - with characteristic peaks expressed in 2-theta ± 0.2 at the following positions: 19.0, 9.9, 12.9, 11.6, 15.9, and 24.2; and/or
   - with characteristic peaks expressed in 2-theta ± 0.2 at the following positions with relative intensities in parenthesis: 19.0 (100.0%), 9.9 (84.2%), 12.9 (36.5%), 11.6 (31.9%), 15.9 (27.6%), and 4.2 (20.3%); and/or
   - substantially the same as that shown in FIG. 2 of WO2013016491; and/or
(c) an endothermic peak in differential scanning calorimetry (DSC) at 60 ± 2 °C.

Methanol solvates of Compound (1) (='Compound (1) • methanol', 'MeOH solvates') are preferably characterized as having
(a) Compound (1) and methanol in a molar ratio of 1: 1 (Compound (1) : methanol); and/or
(b) an X-ray powder diffraction pattern (obtained at room temperature using Cu K alpha radiation)
   - with characteristic peaks expressed in 2-theta ± 0.2 at the following positions: 8.0 and 10.3; and/or
   - with characteristic peaks expressed in 2-theta ± 0.2 at the following positions: 8.0, 10.3, 17.9, 19.9, 20.6, and 22.1; and/or
   - with characteristic peaks expressed in 2-theta ± 0.2 at the following positions with relative intensities in parentheses: 8.0 (100%), 10.3 (68%), 17.9 (59%), 19.9 (63%), 20.6 (39%), and 22.1 (45%); and/or
   - substantially the same as that shown in FIG. 3 of WO2013016491.

Ethanol isopropanol cosolvates of Compound (1) (= 'Compound (1) • ethanol • isopropanol', 'EtOH/IPA solvates') are preferably characterized as having
(a) ethanol and isopropanol in a ratio of 90 : 10 to 97 : 3 (vol %, ethanol : isopropanol);and/or
(b) an X-ray powder diffraction pattern (obtained at room temperature using Cu K alpha radiation)
   - with characteristic peaks expressed in 2-theta ± 0.2 at the following positions: 9.3 and 18.3; and/or
   - with characteristic peaks expressed in 2-theta ± 0.2 at the following positions: 8.7, 9.3, 18.3, 19.8, 15.5, and 23.1; and/or
   - with characteristic peaks expressed in 2-theta ± 0.2 at the following positions with relative intensities in parentheses: 8.7 (18%), 9.3 (77%), 18.3 (100%), 19.8 (24%), 15.5 (45%), and 23.1 (18%); and/or
   - substantially the same as that shown in FIG. 4 of WO2013016491.

Acetone solvates of Compound (1) (='Compound (1) • acetone') are preferably characterized as having
(a) Compound (1) and acetone in a molar ratio of 1: 1 (Compound (1) : acetone)
(b) an X-ray powder diffraction pattern (obtained at room temperature using Cu K alpha radiation)
   - with characteristic peaks expressed in 2-theta ± 0.2 at the following positions: 7.7 and 16.5; and/or
   - with characteristic peaks expressed in 2-theta ± 0.2 at the following positions: 7.7, 10.4, 11.3, 16.5, 19.1, and 21.1; and/or
   - with characteristic peaks expressed in 2-theta ± 0.2 at the following positions with relative intensities in parentheses: 7.7 (100%), 10.4 (63%), 11.3 (43%), 16.5 (69%), 19.1 (37%), and 21.1 (83%); and/or
   - substantially the same as that shown in FIG. 5 of WO2013016491.

Ethylacetate solvates of Compound (1) (='Compound (1) • ethylacetate') are preferably characterized as having
(a) Compound (1) and ethylacetate in a molar ratio of 3 : 1 to 1 : 1 (Compound (1) : ethylacetate); and/or
(b) "ethylacetate solvate A": an X-ray powder diffraction pattern (obtained at room temperature using Cu K alpha radiation)
   - with characteristic peaks expressed in 2-theta ± 0.2 at the following positions: 6.4, 7.3, and 9.8; and/or
   - with characteristic peaks expressed in 2-theta ± 0.2 at the following positions: 6.4, 7.3, 9.8, 17.1, 18.8, and 23.5; and/or
   - with characteristic peaks expressed in 2-theta ± 0.2 at the following positions with relative intensities in parentheses: 6.4 (96%), 7.3 (100%), 9.8 (62%), 17.1 (49%), 18.8 (59%), and 23.5 (47%); and/or
   - substantially the same as that shown in FIG. 6 of WO2013016491 (solvate A); and/or
(c) "ethylacetate solvate B": an X-ray powder diffraction pattern (obtained at room temperature using Cu K alpha radiation)
   - with characteristic peaks expressed in 2-theta ± 0.2 at the following positions: 7.3 an 6.4; and/or
   - with characteristic peaks expressed in 2-theta ± 0.2 at the following positions: 6.4, 7.3, 8.1, 9.0, 18.1, and 19.7; and/or
   - with characteristic peaks expressed in 2-theta ± 0.2 at the following positions with relative intensities in parentheses: 6.4 (29%), 7.3 (100%), 8.1 (27%), 9.0 (8%), 18.1 (15%), 19.7 (9%); and/or
   - substantially the same as that shown in FIG. 7 of WO2013016491 (solvate B); and/or
(d) "ethylacetate solvate C": an X-ray powder diffraction pattern (obtained at room temperature using Cu K alpha radiation)
   - with characteristic peaks expressed in 2-theta ± 0.2 at the following positions: 12.4 and 18.8; and/or
   - with characteristic peaks expressed in 2-theta ± 0.2 at the following positions: 9.7, 11.6, 12.4, 15.7, 18.8, and 23.9; and/or
   - with characteristic peaks expressed in 2-theta ± 0.2 at the following positions with relative intensities in parentheses: 9.7 (100%), 11.6 (17%), 12.4 (13%), 15.7 (34%), 18.8 (15%), and 23.9 (25%); and/or
   - substantially the same as that shown in FIG. 8 of WO2013016491 8 (solvate C); and/or
(e) "ethylacetate solvate D": an X-ray powder diffraction pattern (obtained at room temperature using Cu K alpha radiation)
   - with characteristic peaks expressed in 2-theta ± 0.2 at the following positions: 16.6 and 20.2; and/or
   - with characteristic peaks expressed in 2-theta ± 0.2 at the following positions: 4.9, 9.8, 14.6, 16.6, 20.2, and 21.2; and/or
   - with characteristic peaks expressed in 2-theta ± 0.2 at the following positions with relative intensities in parentheses: 4.9 (43%), 9.8 (71%), 14.6 (48%), 16.6 (100%), 20.2 (91%), and 21.2 (75%); and/or
   - substantially the same as that shown in FIG. 9 of WO2013016491 (solvate D); and/or
(f) "ethylacetate solvate E": an X-ray powder diffraction pattern (obtained at room temperature using Cu K alpha radiation)
   - with characteristic peaks expressed in 2-theta ± 0.2 at the following positions: 12.4 and 18.8; and/or
   - with characteristic peaks expressed in 2-theta ± 0.2 at the following positions: 9.7, 11.6, 12.4, 15.7, 18.8, and 23.9; and/or
   - with characteristic peaks expressed in 2-theta ± 0.2 at the following positions with relative intensities in parentheses: 9.7 (57%), 11.6 (35%), 12.4 (62%), 15.7 (36%), 18.8 (100%), and 23.9 (29%); and/or
   - substantially the same as that shown in FIG. 10 of WO2013016491 (solvate E); and/or
(g) "ethylacetate solvate F": an X-ray powder diffraction pattern (obtained at room temperature using Cu K alpha radiation)
   - with characteristic peaks expressed in 2-theta ± 0.2 at the following positions: 7.3, 17.1, and 22.7; and/or
   - with characteristic peaks expressed in 2-theta ± 0.2 at the following positions: 7.3, 9.7, 17.1, 18.8, 22.7, and 23.5; and/or
   - substantially the same as that shown in FIG. 11 of WO2013016491 (solvate F); and/or
(h) "ethylacetate solvate G": an X-ray powder diffraction pattern (obtained at room temperature using Cu K alpha radiation)
   - with characteristic peaks expressed in 2-theta ± 0.2 at the following positions: 7.5 and 12.1; and/or
   - with characteristic peaks expressed in 2-theta ± 0.2 at the following positions: 7.5, 12.1, 13.0, 13.7, 16.2, and 19.7; and/or
   - substantially the same as that shown in FIG. 12 of WO2013016491 (solvate G).

Isopropylacetate solvates of Compound (1) (='Compound (1) • isopropylacetate', 'IPA solvates') are preferably characterized as having
(a) an X-ray powder diffraction pattern (obtained at room temperature using Cu K alpha radiation)
   - with characteristic peaks expressed in 2-theta ± 0.2 at the following positions: 7.0 and 6.4; and/or
   - with characteristic peaks expressed in 2-theta ± 0.2 at the following positions: 6.4, 7.0, 9.7, 16.1, 18.7, and 22.6.

Ethylacetate 2-methyl THF cosolvates of Compound (1) (='Compound (1) • ethylacetate • 2-methyl THF ') are preferably characterized as having
(a) EtOAc and 2-methyl THF in 70 wt% and 30 wt%; and/or
(b) EtOAc and 2-methyl THF in 90 wt% and 10 wt%; and/or
(c) an X-ray powder diffraction pattern (obtained at room temperature using Cu K alpha radiation)
   - with characteristic peaks expressed in 2-theta ± 0.2 at the following positions: 5.5 and 9.3; and/or
   - with characteristic peaks expressed in 2-theta ± 0.2 at the following positions: 5.5, 9.3, 10.9, 12.6, 17.7, and 19.9; and/or
   - substantially the same as that shown in FIG. 13 of WO2013016491; and/or
(d) an X-ray powder diffraction pattern (obtained at room temperature using Cu K alpha radiation)
   - with characteristic peaks expressed in 2-theta ± 0.2 at the following positions: 6.5 and 7.3; and/or
   - with characteristic peaks expressed in 2-theta ± 0.2 at the following positions: 6.5, 7.3, 9.1, 17.0, 18.7, and 23.5; and/or
   - substantially the same as that shown in FIG. 14 of WO2013016491.

Ethanol solvates of Compound (1) (='Compound (1) • ethanol') are preferably characterized as having
(a) Compound (1) and ethanol in a molar ratio of 1: 1 (Compound (1): ethanol); and/or
(b) an X-ray powder diffraction pattern (obtained at room temperature using Cu K alpha radiation)
   - with characteristic peaks expressed in 2-theta ± 0.2 at the following positions: 8.1 and 10.1; and/or
   - with characteristic peaks expressed in 2-theta ± 0.2 at the following positions: 8.1, 10.1, 18.1, 19.6, 21.7, and 25.6; and/or
   - substantially the same as that shown in FIG. 15 of WO2013016491.

n-Butylacetate solvates of Compound (1) (='Compound (1) • n-butylacetate') are preferably characterized as having
(a) Compound (1) and n-butylacetate in a molar ratio of 4: 1 - 1: 1 (Compound (1) • n-butylacetate); and/or
(b) "n-butylacetate solvate A": an X-ray powder diffraction pattern (obtained at room temperature using Cu K alpha radiation)
   - with characteristic peaks expressed in 2-theta ± 0.2 at the following positions: 9.7 and 16.5; and/or
   - with characteristic peaks expressed in 2-theta ± 0.2 at the following positions: 9.7, 14.9, 16.5, 19.6, 20.0, and 21.0; and/or
   - substantially the same as that shown in FIG. 16 of WO2013016491; and/or
(c) "n-butylacetate solvate B": an X-ray powder diffraction pattern (obtained at room temperature using Cu K alpha radiation)
   - with characteristic peaks expressed in 2-theta ± 0.2 at the following positions: 6.4 and 6.9; and/or
   - with characteristic peaks expressed in 2-theta ± 0.2 at the following positions: 6.4, 6.9, 17.5, 18.2, 18.9, and 23.2; and/or
   - substantially the same as that shown in FIG. 17 of WO2013016491; and/or
(d) "n-butylacetate solvate C": an X-ray powder diffraction pattern (obtained at room temperature using Cu K alpha radiation)
   - with characteristic peaks expressed in 2-theta ± 0.2 at the following positions: 6.9 and 9.6; and/or
   - with characteristic peaks expressed in 2-theta ± 0.2 at the following positions: 6.9, 9.6, 15.9, 16.9, 18.6, and 19.3; and/or
   - substantially the same as that shown in FIG. 18 of WO2013016491.

Heptane solvates of Compound (1) (='Compound (1) • heptane') are preferably characterized as having
(a) "heptane solvate A": an X-ray powder diffraction pattern (obtained at room temperature using Cu K alpha radiation)
   - with characteristic peaks expressed in 2-theta ± 0.2 at the following positions: 10.7 and 21.3; and/or
   - with characteristic peaks expressed in 2-theta ± 0.2 at the following positions: 10.7, 12.3, 14.0, 17.2, 19.6, and 21.3; and/or
   - substantially the same as that shown in FIG. 19 of WO2013016491; and/or
(b) "heptane solvate B": an X-ray powder diffraction pattern (obtained at room temperature using Cu K alpha radiation)
   - with characteristic peaks expressed in 2-theta ± 0.2 at the following positions: 5.5 and 7.5; and/or
   - with characteristic peaks expressed in 2-theta ± 0.2 at the following positions: 5.5, 7.5, 9.3, 10.9, 16.5, and 22.1; and/or
   - substantially the same as that shown in FIG. 20 of WO2013016491; and/or
(c) "heptane solvate C": an X-ray powder diffraction pattern (obtained at room temperature using Cu K alpha radiation)
   - with characteristic peaks expressed in 2-theta ± 0.2 at the following positions: 10.4 and 11.2; and/or
   - with characteristic peaks expressed in 2-theta ± 0.2 at the following positions: 10.4, 11.2, 13.4, 16.9, 19.3, and 19.8; and/or
   - substantially the same as that shown in FIG. 21 of WO2013016491; and/or
(d) "heptane solvate D": an X-ray powder diffraction pattern (obtained at room temperature using Cu K alpha radiation)
   - with characteristic peaks expressed in 2-theta ± 0.2 at the following positions: 10.3 and 13.7; and/or
   - with characteristic peaks expressed in 2-theta ± 0.2 at the following positions: 10.3, 13.7, 16.9, 18.9, 19.3, and 20.4; and/or
   - substantially the same as that shown in FIG. 22 of WO2013016491; and/or
(e) "heptane solvate E": an X-ray powder diffraction pattern (obtained at room temperature using Cu K alpha radiation)
   - with characteristic peaks expressed in 2-theta ± 0.2 at the following positions: 5.0 and 10.2; and/or
   - with characteristic peaks expressed in 2-theta ± 0.2 at the following positions: 5.0, 10.2, 16.9, 19.3, 20.5, and 21.5; and/or
   - substantially the same as that shown in FIG. 23 of WO2013016491.

2-Butanone (methylethylketone (MEK)) solvates of Compound (1) (= 'Compound (1) • 2-butanone', 'MEK solvates') are preferably characterized as having
(a) a solid state C¹³ nuclear magnetic spectroscopy (NMR) spectrum
   - having peaks at: 205.7, 132.5, 127.7, 42.9, and 37.4; and/or
   - substantially the same as those shown in FIG. 24 of WO2013016491.

Methylacetate solvates of Compound (1) (='Compound (1) • methylacetate') are preferably characterized as having
(a) a solid state C¹³ nuclear magnetic spectroscopy (NMR) spectrum
   - having peaks at: 170.5, 137.2, 106.5, 54.9, and 51.1; and/or
   - substantially the same as those shown in FIG. 25 of WO2013016491.

### SPECIFIC INORMATION CONCERNING ASPECT (I) OF THE INVENTION

Aspect (i) of the invention concerns amorphous form of Compound (1) as well as polymorphic forms of Compound (1) and relates to administration units comprising amorphous Form, polymorphic Form M, polymorphic Form H, polymorphic Form P, polymorphic Form X, or polymorphic Form ZA, respectively. The invention relates to a solid form of Compound (1), namely to an amorphous or a polymorphic form of Compound (1), which is useful for treating inhibiting or reducing the activity of HCV polymerase in a biological in vitro sample or in a subject, and of treating a HCV infection in a subject. Polymorphic forms of Compound (1) are described in WO2013016490, which is incorporated by reference.

Compound (1) is represented by the following structural formula:

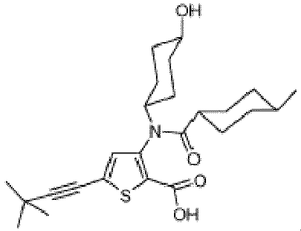

Compound (1) and pharmaceutically acceptable salts thereof are NS5B polymerase inhibitors, and also described in WO 2008/058393. As used herein, amorphous form of Compound (1) is a solid form of Compound (1) and polymorphic forms of Compound (1) are crystalline forms of Compound (1) characterized as described in WO2013016490 as follows:
The present invention generally relates to polymorphic forms of Compound (1), to methods of inhibiting or reducing the activity of HCV polymerase in a biological in vitro sample or in a subject, and of treating a HCV infection in a subject, which employ the to polymorphic forms of Compound (1), and to methods of preparing such forms.

In one embodiment, the present invention is directed to polymorph Form M of Compound (1). In another embodiment, the present invention is directed to polymorph Form H of Compound (1). In yet another embodiment, the present invention is directed to polymorph Form P of Compound (1). In yet another embodiment, the present invention is directed to amorphous form of Compound (1). In yet another embodiment, the present invention is directed to polymorph Form X of Compound (1). In yet another embodiment, the present invention is directed to polymorph Form ZA of Compound (1). In yet another embodiment, the present invention is directed to a pharmaceutical composition comprising: a polymorphic form selected from the group consisting of Form M, Form H, and Form P of Compound (1); or amorphous form of Compound (1), and at least one pharmaceutically acceptable carrier or excipient. In yet another embodiment, the present invention is directed to a pharmaceutical composition comprising: a polymorphic form selected from the group consisting of Form X and Form ZA of Compound (1); and at least one pharmaceutically acceptable carrier or excipient. In yet another embodiment, the present invention is directed to a method of inhibiting or reducing the activity of HCV polymerase in a biological in vitro sample. The method includes administering to the sample an effective amount of: a polymorphic form selected from the group consisting of Form M, Form H, Form P, Form X, and Form ZA of Compound (1); or amorphous form of Compound (1). In yet another embodiment, the present invention is directed to a method of inhibiting or reducing the activity of HCV polymerase in a subject. The method includes administering to the subject an effective amount of: a polymorphic form selected from the group consisting of Form M, Form H, Form P, Form X, and Form ZA of Compound (1); or amorphous form of Compound (1). In yet another embodiment, the present invention is directed to a method of treating a HCV infection in a subject. The method includes administering to the subject an effective amount of: a polymorphic form selected from the group consisting of Form M, Form H, Form P, Form X, and Form ZA of Compound (1); or amorphous form of Compound (1). Methods of preparing polymorph Forms M, H, P, X and ZA of Compound (1) are also provided. A method of preparing polymorph Form M of Compound (1) includes stirring a mixture of Compound (1) and a solvent system that includes isopropanol, ethyl acetate, n-butyl acetate, methyl acetate, acetone, 2-butanone (methylethylketone (MEK)), or heptane, or a combination thereof at a temperature in a range of 10 °C to 47 °C to form From M of Compound (1). A method of preparing polymorph Form H of Compound (1) includes stirring a solution of Compound (1) at a temperature in a range of 48 °C to 70 °C to form Form H of Compound (1). A method of preparing polymorph Form P of Compound (1) includes stirring a mixture of Compound (1) and a solvent system that includes a solvent selected from the group consisting of dichloromethane, tetrahydrofuran (THF), and a mixture thereof at room temperature to form Form P of Compound (1). A method of preparing polymorph Form X of Compound (1) includes removing ethyl acetate from ethylacetate solvate G of Compound (1), wherein ethylacetate solvate G of Compound (1) is characterized as having an X-ray powder diffraction pattern with characteristic peaks expressed in 2-theta ± 0.2 at the following positions: 7.5 and 12.1, wherein the X-ray powder diffraction pattern is obtained at room temperature using Cu K alpha radiation. A method of preparing polymorph Form ZA of Compound (1) includes removing n-butyl acetate from n-butyl acetate solvate A of Compound (1), wherein n-butyl acetate solvate A of Compound (1) is characterized as having an X-ray powder diffraction pattern with characteristic peaks expressed in 2-theta ± 0.2 at the following positions: 9.7 and 16.5, wherein the X-ray powder diffraction pattern is obtained at room temperature using Cu K alpha radiation. Use of the polymorphic forms of Compound (1) described herein for inhibiting or reducing the activity of HCV polymerase in a biological in vitro sample or in a subject is also provided. Use of the polymorphic forms of Compound (1) for treating a HCV infection in a subject is also provided. Also provided herein is use of amorphous form of Compound (1) for inhibiting or reducing the activity of HCV polymerase in a biological in vitro sample or in a subject. Use of amorphous Compound (1) for treating a HCV infection in a subject is also provided. The present invention also provides use of the polymorphic forms of Compound (1) or amorphous Compound (1) described herein in the manufacture of a medicament for treating a HCV infection in a subject.

FIGs. 1-4 of WO2013016490 show room temperature XRPD patterns of Form A, Form M, Form H, and Form P of Compound (1), respectively. FIGs. 5-8 of WO2013016490 show solid state C¹³ nuclear magnetic spectroscopies (SSNMR) of Form A, Form M, Form H, and Form P of Compound (1), respectively. FIG. 9 of WO2013016490 shows solid state C¹³ nuclear magnetic spectroscopy (SSNMR) of amorphous Compound (1). FIGs. 10 and 11 of WO2013016490 show room temperature XRPD patterns of Form X and Form ZA of Compound (1), respectively.

Compound (1) represented by the following structural formula: 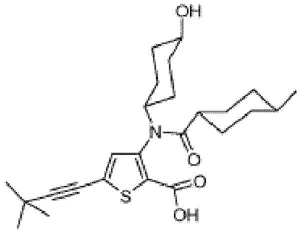 and pharmaceutically acceptable salts thereof are NS5B polymerase inhibitors, and also described in WO 2008/058393. Compound (1) can exist in different polymorphic forms. As known in the art, polymorphism is an ability of a compound to crystallize as more than one distinct crystalline or "polymorphic" species. A polymorph is a solid crystalline phase of a compound with at least two different arrangements or polymorphic forms of that compound molecule in the solid state. Polymorphic forms of any given compound are defined by the same chemical formula or composition and are as distinct in chemical structure as crystalline structures of two different chemical compounds. Generally, different polymorphs can be characterized by analytical methods such as X-ray powder diffraction (XRPD) pattern, thermogravimetric analysis (TGA), and differential scanning calorimetry (DSC), or by its melting point, or other techniques known in the art. As used herein, the term "polymorphic form" means a neat polymorphic form that does not have any solvates.

In a preferred embodiment, the present invention is directed to methods of preparing Form M, Form H, Form P, Form X, and Form ZA of Compound (1). Form M of Compound (1) can be prepared by a method employing stirring a mixture of Compound (1) and a solvent system that includes isopropanol, ethyl acetate, n-butyl acetate, methyl acetate, acetone, 2-butanone, or heptane, or a combination thereof at a temperature in a range of 10 °C to 47 °C to form From M of Compound (1). In one specific embodiment, the solvent system includes: isopropanol; ethylacetate; n-butylacetate; a mixture of n-butylacetate and acetone (e.g, 5 wt% -95 wt% of n-butylacetate and 5 wt% -95 wt% of acetone, such as 90 wt% of n-butylacetate and 10 wt% of acetone); a mixture of n-butylacetate and methylacetate (e.g, 5 wt% -95 wt% of n-butylacetate and 5 wt% -95 wt% of methylacetate, such as 50 wt% of n-butylacetate and 50 wt% of methylacetate); acetone; 2-butanone (methylethylketone (MEK)); a mixture of n-butylacetate and heptane (e.g, 5 wt% -95 wt% of n-butylacetate and 5 wt% -95 wt% of heptane, such as 50 wt% of n-butylacetate and 50 wt% of heptane); a mixture of acetone and heptane (e.g, 5 wt% -95 wt% of acetone and 5 wt% -95 wt% of heptane, such as 50 wt% of acetone and 50 wt% of heptane); or a mixture of ethylacetate and heptane (e.g, 5 wt% -95 wt% of ethylacetate and 5 wt% -95 wt% of heptane, such as 50 wt% of ethylacetate and 50 wt% of heptane). In another specific embodiment, Form M of Compound (1) can be prepared by employing stirring Compound (1): i) in isopropanol at a temperature in a range of 10 °C to 47 °C; ii) in ethyl acetate at a temperature in a range of 45 °C to 47 °C; iii) in n-butyl acetate at a temperature in a range of 35 °C to 47 °C; iv) in a mixture of n-butylacetate and acetone (e.g, 5 wt% -95 wt% of butylacetate and 5 wt% -95 wt% of acetone, such as 90 wt% of butylacetate and 10 wt% of acetone) at a temperature in a range of 30 °C to 47 °C; v) in a mixture of n-butylacetate and methylacetate (e.g, 5 wt% -95 wt% of n-butylacetate and 5 wt% -95 wt% of methylacetate, such as 50 wt% of n-butylacetate and 50 wt% of methylacetate) at a temperature in a range of 25 °C to 47 °C; vi) in acetone at a temperature in a range of 20 °C to 47 °C; vii) in 2-butanone (MEK) at a temperature in a range of 30 °C to 47 °C; viii) in a mixture of n-butyl acetate and heptane (e.g, 5 wt% -95 wt% of n-butylacetate and 5 wt% -95 wt% of heptane, such as 50 wt% of n-butylacetate and 50 wt% of heptane) at a temperature in a range of 25 °C to 47 °C; ix) in a mixture of acetone and heptane (e.g, 5 wt% -95 wt% of acetone and 5 wt% -95 wt% of heptane, such as 50 wt% of acetone and 50 wt% of heptane) at a temperature in a range of 25 °C to 47 °C; x) or in a mixture of ethylacetate and heptane (e.g, 5 wt% -95 wt% of ethylacetate and 5 wt% -95 wt% of heptane, such as 50 wt% of ethylacetate and 50 wt% of heptane) at a temperature in a range of 25 °C to 47°C.

Form H of Compound (1) can be prepared by a method employing stirring a solution of Compound (1) at a temperature in a range of 48 °C to 70 °C, such as at a temperature in a range of 50 °C to 70 °C or 55 °C to 70 °C. In one specific embodiment, a mixture of Compound (1) and a solvent system that includes ethylacetate is stirred at a temperature in a range of 50 °C to 70 °C for a period of time to form Form H. In another specific embodiment, a mixture of Compound (1) and a solvent system that includes ethyl acetate is stirred at a temperature in a range of 55 °C to 70 °C for a period of time to form Form H. In yet another specific embodiment, a mixture of Compound (1) and a solvent that includes ethylacetate is stirred at a temperature of 65 ± 2 °C for a period of time to form Form H.

Form P of Compound (1) can be prepared by a method employing heating a mixture of Compound (1) and a solvent system that includes a solvent selected from the group consisting of dichloromethane, and tetrahydrofuran (THF), and a mixture thereof at room temperature. In one specific embodiment, the mixture of Compound (1) and a solvent system that includes dichloromethane is stirred at room temperature for a period of time to form Form P.

Form X of Compound (1) can be prepared by a method employing removing ethyl acetate from ethylacetate solvate G of Compound (1). Typically, ethylacetate solvate G of Compound (1) is characterized as having an X-ray powder diffraction pattern with characteristic peaks expressed in 2-theta ± 0.2 at the following positions: 7.5 and 12.1, wherein the X-ray powder diffraction pattern is obtained at room temperature using Cu K alpha radiation. In one specific embodiment, the ethylacetate solvate G is further characterized as having an X-ray powder diffraction pattern with characteristic peaks expressed in 2-theta ± 0.2 at the following positions: 7.5, 12.1, 13.0, 13.7, 16.2, and 19.7, wherein the X-ray powder diffraction pattern is obtained at room temperature using Cu K alpha radiation.

Form ZA of Compound (1) can be prepared by a method employing removing n-butylacetate from n-butylacetate solvate A of Compound (1). Typically, n-butylacetate solvate A of Compound (1) is characterized as having an X-ray powder diffraction pattern with characteristic peaks expressed in 2-theta ± 0.2 at the following positions: 9.7 and 16.5, wherein the X-ray powder diffraction pattern is obtained at room temperature using Cu K alpha radiation. In one specific embodiment, n-butylacetate solvate A is further characterized as having an X-ray powder diffraction pattern with characteristic peaks expressed in 2-theta ± 0.2 at the following positions: 9.7, 14.9, 16.5, 19.6, 20.0, and 21.0, wherein the X-ray powder diffraction pattern is obtained at room temperature using Cu K alpha radiation.

Typically, solvates of Compound (1) can be prepared by stirring a mixture of Compound (1) and a desired solvent at a suitable temperature (e.g., room temperature, 10 °C - 35 °C, or 20 °C - 25 °C) for a sufficient time to form the desired solvates of Compound (1). For example, the ethylacetate solvates of Compound (1) can be prepared by stirring a mixture of Compound (1) and ethylacetate at a temperature in a range of 5 °C to 50 °C (e.g., 5 °C to 35 °C or 10 °C to 50 °C) and the n-butylacetate solvates of Compound (1) can be prepared by stirring a mixture of Compound (1) and n-butylacetate at room temperature.

In yet another embodiment, the present invention is directed to methods of preparing amorphous Compound (1). Amorphous Compound (1) can be prepared by employing spray drying a solution of crystalline Compound (1). In one specific embodiment, the crystalline Compound (1) is Form A, Form M, Form P, or From H. In yet another specific embodiment, the crystalline Compound (1) is Form A. In yet another specific embodiment, the methods employ spray drying a solution of crystalline Compound (1) in ethanol. Any suitable conditions for spray drying can be employed in the invention. Specific exemplary conditions are described below in the Exemplification section.

The present invention encompasses the polymorphic forms of Compound (1) and amorphous Compound (1) described above in isolated, pure form, or in a mixture as a solid composition when admixed with other materials, for example the other known polymorphic forms (i.e. amorphous form, Form A of Compound (1), or other forms) of Compound (I) or any other materials.

Thus in one aspect there are provided polymorphic Forms M, H, P, X, and ZA of Compound (1) in isolated solid form. In another aspect there is provided amorphous Compound (1) in isolated solid form.

In a further aspect there are provided polymorphic Forms M, H, P, X, and ZA of Compound (1) in pure form. The pure form means that Form M, H, P, X, and ZA of Compound (1) is over 95% (w/w), for example, over 98% (w/w), over 99% (w/w %), over 99.5% (w/w), or over 99.9%) (w/w). In another further aspect there is provided amorphous Compound (1) in pure form. The pure form means that amorphous Compound (1) is over 95% (w/w), for example, over 98% (w/w), over 99% (w/w %), over 99.5% (w/w), or over 99.9% (w/w).

More specifically, the present invention provides that each of polymorphic Forms M, H, P, X, and ZA of Compound (1) is in the form of a composition or a mixture of the polymorphic form with one or more other crystalline, solvate, amorphous, or other polymorphic forms or their combinations thereof. For example, such a composition may comprise polymorphic Form M along with one or more other polymorphic forms of Compound (1), such as amorphous form, hydrate, solvates, polymorph Form A, Form H, Form P, and/or other forms or their combinations thereof. Similarly, such a composition may comprise polymorphic Form H along with one or more other polymorphic forms of Compound (1), such as amorphous form, hydrate, solvates, polymorph Form A, Form M, Form P, and/or other forms or their combinations thereof. Also, such a composition may comprise polymorphic Form P along with one or more other polymorphic forms of Compound (1), such as amorphous form, hydrate, solvates, polymorph Form A, Form M, Form H, and/or other forms or their combinations thereof. More specifically, the composition may comprise from trace amounts up to 100% polymorphic Forms M, H, or P of Compound (1), or any amount in between- for example, in a range of 0.1 % - 0.5%, 0.1 % - 1%, 0.1% - 2%, 0.1% - 5%, 0.1% - 10%, 0.1% - 20%, 0.1% - 30%, 0.1% - 40%, or 0.1% - 50% by weight based on the total amount of Compound (1) in the composition. Alternatively, the composition may comprise at least 50%, 60%, 70%, 80%, 90%, 95%, 97%, 98%, 99%, 99.5% or 99.9% by weight of polymorphic Forms M, H, or P of Compound (1) based on the total amount of Compound (1) in the composition

The experimental section of WO2013016490 contains the following information:

### Example 3: Formation of Polymorphic Forms of Compound (1)

### 3A: Formation of Polymorphic Form A of Compound (1)

Polymorphic Form A of Compound (1) can be prepared by following the steps described below: 10 g of Compound (1) was charged to a reactor. 20 g of methanol was then charged to the reactor. The reactor was heated to 60 °C to dissolve Compound (1). The reactor was then cooled to 10 °C, and left until solids of Compound (1) formed. The solids of Compound (1) were filtered. 20 g of acetone at 25 °C was added to the solids of Compound (1). The mixture of acetone and Compound (1) was stirred for 1 hour and the resulting solids were filtered. The filtered solids were dried at 75 °C for 12 hours. Characteristics of Form A of Compound (1): XRPD data and C¹³ solid state NMR data of Form A of Compound (1) are shown in FIG. 1 of WO2013016490 and FIG. 5 of WO2013016490, respectively. Certain representative XRPD peaks and DSC endotherm (°C) of Form A of Compound (1) are summarized in Table 1 below.

**Table 1: Certain representative XRPD Peaks and DSC Endotherm of Form A**

| | **Form A** | |
|---|---|---|
| **DSC Endotherm (°C)** | 188 °C | |
| | | |

| **XRPD Peaks** | **Angle (2-Theta ± 0.2)** | **Intensity %** |
|---|---|---|
| 1 | 6.9 | 100.0 |
| 2 | 16.6 | 53.3 |
| 3 | 21.7 | 31.6 |
| 4 | 8.6 | 31.3 |
| 5 | 11.6 | 26.2 |
| 6 | 19.4 | 23.8 |

### 3B: Formation of Polymorphic Form M of Compound (1)

1. Method A: Polymorphic Form M of Compound (1) can be prepared by following the steps described below: 10 g of Compound (1) was charged to a reactor. 50 g of ethyl acetate was then charged to the reactor. The reactor was heated to 45 °C and the mixture was stirred for 1 - 2 days until Form M was observed. Then, the reactor was cooled to 25 °C, and left until solids of Compound (1) formed. The solids of Compound (1) were filtered and the filtered solids were dried at 35 °C for 24 hours.
2. Method B: Polymorphic Form M of Compound (1) was also be prepared in a similar manner as described above for Method A but employing a solvent system listed in Table 2A below and stirring Compound (1) in the solvent system at a respective temperature range listed in Table 2A.

**Table 2A: Conditions for the Preparation of Form M**

| **Solvents** | **Form M Temperature Window** |
|---|---|
| n-BuOAc | 35-47°C |
| n-BuOAc/Acetone (90%/10%, w/w) | 30-47°C |
| n-BuOAc/(50%/50%, w/w) | 25-47°C |
| Acetone | 20-47°C |
| MEK | 30-47°C |
| n-BuOAc /Heptane (50%/50%, w/w) | 25-47°C |
| Acetone / Heptane (50%/50%, w/w) | 25-47°C |
| EtOAc/Heptane (50%/50%, w/w) | 25-47°C |
| EtOAc | 45-47°C |

Characteristics of Form M of Compound (1): XRPD data and C solid state NMR data of Form M of Compound (1) are shown in FIG. 2 of WO2013016490 and FIG. 6 of WO2013016490, respectively. Certain representative XRPD peaks and DSC endotherm (°C) of Form M of Compound (1) are summarized in Table 2B below.

**Table 2B: Certain representative XRPD Peaks and DSC Endotherm of Form M**

| | **Form M** | |
|---|---|---|
| **DSC Endotherm** (°C) | 230°C | |
| | | |

| **XRPD Peaks** | **Angle (2-Theta ± 0,2)** | **Intensity %** |
|---|---|---|
| 1 | 19.6 | 100.0 |
| 2 | 16.6 | 72.4 |
| 3 | 18.1 | 59.8 |
| 4 | 9.0 | 47.6 |
| 5 | 22.2 | 39.9 |
| 6 | 11.4 | 36.6 |

### 3C: Formation of Polymorphic Form H of Compound (1)

Polymorphic Form H of Compound (1) can be prepared by following the steps described below: 10 g of Compound (1) was charged to a reactor. 50 g of ethyl acetate was then charged to the reactor. The reactor was heated to 65 °C and the mixture was stirred for 1 - 2 days until Form H was observed. If desired, a seed(s) of Form H could be added into the reactor for a large scale production. Then, the reactor was cooled to 25 °C, and left until solids of Compound (1) formed. The solids of Compound (1) were filtered and the filtered solids were dried at 65 °C for 24 hours. Characteristics of Form H of Compound (1): XRPD data and C¹³ solid state NMR data of Form H of Compound (1) are shown in FIG. 3 of WO2013016490 and FIG. 7 of WO2013016490, respectively. Certain representative XRPD peaks and DSC endotherm (°C) of Form H of Compound (1) are summarized in Table 3 below.

**Table 3: Certain representative XRPD Peaks and DSC Endotherm of Form H**

| | **Form H** | |
|---|---|---|
| **DSC Endotherm (°C)** | 238°C | |
| | | |

| **XRPD Peaks** | **Angle (2-Theta ± 0.2)** | **Intensity %** |
|---|---|---|
| 1 | 6.6 | 100.0 |
| 2 | 18.7 | 87.8 |
| 3 | 8.5 | 66.7 |
| 4 | 17.3 | 58.4 |
| 5 | 15.8 | 39.9 |
| 6 | 19.4 | 29.8 |

### 3D: Formation of Polymorphic Form P of Compound (1)

Polymorphic Form P of Compound (1) can be prepared by following the steps described below:
Method A: 20 mg of Compound (1) was charged to a vial. 0.5 mL of dicholormethane was then charged to the vial. The mixture was stirred at room temperature for 3 weeks until solids of Compound (1) were formed. The solids of Compound (1) were filtered and the filtered solids were dried at room temperature for 1 hour.
Method B: 500 mg of Compound (1) was charged to a vial. 6 mL of dicholormethane was then charged to the vial. The mixture was stirred at room temperature for 4 days until solids of Compound (1) were formed. The solids of Compound (1) were filtered and the filtered solids were dried at room temperature for 1 hour. Characteristics of Form P of Compound (1): XRPD data and C¹³ solid state NMR data of Form P of Compound (1) are shown in FIG. 4 of WO2013016490 and FIG. 8 of WO2013016490, respectively. Certain representative XRPD peaks and DSC endotherm (°C) of Form P of Compound (1) are summarized in Table 4 below.

**Table 4: Certain representative XRPD Peaks and DSC Endotherm of Form P**

| | **Form P** | |
|---|---|---|
| **DSC Endotherm (°C)** | 160 °C | |
| | | |
| | | |

| **XRPD Peaks** | **Angle (2-Theta ± 0.2)** | **Intensity %** |
|---|---|---|
| 1 | 7.0 | 100.0 |
| 2 | 15.8 | 21.9 |
| 3 | 9.8 | 14.6 |
| 4 | 19.3 | 11.9 |
| 5 | 8.5 | 10.5 |
| 6 | 21.9 | 9.5 |

### 3E: Formation of Polymorphic Form X of Compound (1)

Polymorphic Form X of Compound (1) can be prepared by following the steps described below: 50 mg of EtOAc Solvate G was placed into an open 20 mL vial in a vacuum oven at 60°C for 24 hours. After 24 hours the vial was removed and the powder was analyzed by XRPD. Form X was isostructural with EtOAc Solvate G so the location of the peaks listed in the xrpd patterns were within 0.2 degrees 2-theta of each other. Characteristics of Form X of Compound (1): XRPD data of Form X of Compound (1) are shown in FIG. 10 of WO2013016490. Certain representative XRPD peaks of Form X of Compound (1) are summarized in Table 5 below.

**Table 5: Certain representative XRPD Peaks of Form X**

| | **Form P** |
|---|---|
| **XRPD Peaks** | **Angle (2-Theta ± 0.2)** |
| 1 | 7.5 |
| 2 | 12.1 |
| 3 | 13.0 |
| 4 | 13.8 |
| 5 | 16.2 |
| 6 | 19.7 |

### 3F: Formation of Polymorphic Form ZA of Compound (1)

Polymorphic Form ZA of Compound (1) can be prepared by following the steps described below: 3 mg of n-BuOAc solvate A of Compound (1) was placed into an aluminum DSC pan. The sample was heated at a rate of 10°C per minute to 145°C to remove n-BuOAc from n-BuOAc solvate A. Characteristics of Form ZA of Compound (1): XRPD data of Form ZA of Compound (1) are shown in FIG. 11 of WO2013016490. Certain representative XRPD peaks of Form ZA of Compound (1) are summarized in Table 6 below.

**Table 6: Certain representative XRPD Peaks of Form ZA**

| | **Form ZA** |
|---|---|
| **XRPD Peaks** | **Angle (2-Theta ± 0.2)** |
| 1 | 5.2 |
| 2 | 10.2 |
| 3 | 16.5 |
| 4 | 18.6 |
| 5 | 19.8 |
| 6 | 20.3 |

### 3G: Formation of Amorphous Compound (1)

Spray dried amorphous Compound (1) was developed by dissolving crystalline drug substance (Compound (1): Form A) in a processing solvent (Ethanol) at approximately 10% w/w solids load. This solution was spray dried using a Buchi mini spray dryer (B-290), setup in a closed loop configuration, using a Buchi condenser (B-295) to condense the solvent (ethanol) from the exhaust nitrogen.

Solution Preparation: 20g of Compound (1) was dissolved in 180g of ethanol as shown in Table 7.

**Table 7: Materials Used in Preparation of Solution Processed to Manufacture Amorphous Compound (1)**

| **Material** | **Amount (g)** |
|---|---|
| Crystalline Compound **(1)** Form A | 20.00 |
| Ethanol | 180.07 |
| | 200.07 |

| | |
|---|---|
| *The solvent is removed during spray drying process. | |

Spray Drying Procedure: The final solution was then spray dried using the Buchi B-290 mini spray dryer at the spray settings shown in Table 8.

**Table 8: Spray Drying Settings**

| **Processing Parameter** | **Setting** |
|---|---|
| Inlet Temperature | 135°C |
| Outlet Temperature | 50°C |
| Nitrogen pressure | 120psi |
| Aspirator | 100% |
| Solution Pump | 35% |
| Rota meter | 40 mm |
| | |
| Filter pressure | 20 mbar |
| Chiller Temperature | -20°C |

The resulting amorphous material was tray dried at 40°C for 24hrs to remove any residual ethanol solvent. The dry amorphous material was collected and tested for amorphous content using particle x-ray diffraction on the Bruker D8 Discover. XRPD data confirmed that the material prepared was amorphous. FIG. 9 of WO2013016490 shows solid state C¹³ nuclear magnetic spectroscopy (SSNMR) of amorphous Compound (1).

### Example 4: Preparation of Various Solvates of Compound (1)

4A: Formation of Methanol Solvates of Compound (1) (Compound (1) • MeOH): Methanol solvates of Compound (1) can be prepared by following the steps described below: A slurry 20 mg of Compound (1) in 500 microliters of MeOH was stirred at room temperature for 3 weeks in a capped HPLC vial to form Compound (1)"MeOH. The solids were collected by filtration and analyzed by XRPD. TGA data indicated a methanol solvate with a stoichiometry of approximately 1: 1 (Compound (1) • methanol). Characteristics of methanol solvates of Compound (1): Certain representative XRPD peaks of methanol solvates of Compound (1) are summarized in Table 9 below.

**Table 9: Certain representative XRPD of Methanol Solvates of Compound (1)**

| | **Methanol Solvates** | |
|---|---|---|
| **XRPD Peaks** | **Angle (2-Theta ± 0.2)** | **Intensity %** |
| 1 | 8.0 | 100 |
| 2 | 10.3 | 68 |
| 3 | 17.9 | 59 |
| 4 | 19.9 | 63 |
| 5 | 20.6 | 39 |
| 6 | 22.1 | 45 |

4B: Formation of Ethylacetate Solvates of Compound (1) (Compound (1) • EtOAc): EtOAc solvates A-F of Compound (1) (Compound (1) • EtOAc) can be prepared by following the steps described below: 1. EtOAc solvate A: A slurry containing 100 mg of Compound (1) in EtOAc in a 2 mL vial was stirred at room temperature overnight. The solvent was decanted off giving the remaining wet-cake which was analyzed by XRPD. TGA data indicated an EtOAc solvate with a stoichiometry of approximately 3: 1 (Compound (1) • EtOAc). Characteristics of EtOAc solvate A: Certain representative XRPD peaks of EtOAc solvate A are summarized in Table 10 below.

**Table 10: Certain representative XRPD of EtOAc solvate A**

| | EtOAc solvate A | |
|---|---|---|
| **XRPD Peaks** | **Angle (2-Theta ± 0.2)** | **Intensity %** |
| 1 | 6.4 | 96 |
| 2 | 7.0 | 100 |
| 3 | 9.7 | 62 |
| 4 | 16.1 | 49 |
| 5 | 18.7 | 59 |
| 6 | 22.6 | 47 |

2. EtOAc solvate B: A slurry containing 20 mg of Compound (1) in 500 microliters of EtOAc in a capped vial was stirred at room temperature for 3 weeks. The solids were collected by filtration and analyzed by XRPD. Characteristics of EtOAc solvate B: Certain representative XRPD peaks of EtOAc solvate B are summarized in Table 11 below.

**Table 11: Certain representative XRPD of EtOAc solvate B**

| | EtOAc B |
|---|---|
| **XRPD Peaks** | **Angle (2-Theta ± 0.2)** |
| 1 | 6.4 |
| 2 | 7.3 |
| 3 | 8.1 |
| 4 | 9.0 |
| 5 | 18.1 |
| 6 | 19.7 |

3. EtOAc solvate C: Approximately 20 kg of Compound (1) was added to a reactor. 200 kg of 2-MeTHF was then charged to the reactor. 200 kg of EtOAc was then added to the reactor and the solution was rotovapped at 100 mmHg and 30°C which resulted in oil being obtained. The reactor was then charged with 591 kg of EtOAc which was then rotovapped at 50 mmHg and 30°C. The solid residue was submitted for XRPD. Characteristics of EtOAc solvate C: Certain representative XRPD peaks of EtOAc solvate C are summarized in Table 12 below.

**Table 12: Certain representative XRPD of EtOAc solvate C**

| | EtOAc solvate C | |
|---|---|---|
| **XRPD Peaks** | **Angle (2-Theta** ± **0.2)** | **Intensity %** |
| 1 | 6.3 | 100 |
| 2 | 14.6 | 17 |
| | | |
| 3 | 15.6 | 13 |
| 4 | 18.9 | 34 |
| 5 | 20.4 | 15 |
| 6 | 22.2 | 25 |

4. EtOAc solvate D: 550 mg of Compound (1) was added to 2 mL of EtOAc. The slurry was shaken for 4 days at 400 rpm between 20°C and 25°C. The sample was then filtered and analyzed for XRPD. Characteristics of EtOAc solvate D: Certain representative XRPD peaks of EtOAc solvate D are summarized in Table 13 below.

**Table 13: Certain representative XRPD of EtOAc solvate D**

| | EtOAc solvate D | |
|---|---|---|
| **XRPD Peaks** | **Angle (2-Theta ± 0.2)** | **Intensity %** |
| 1 | 4.9 | 43 |
| 2 | 9.8 | 71 |
| 3 | 14.6 | 48 |
| 4 | 16.6 | 100 |
| 5 | 20.2 | 91 |
| 6 | 21.2 | 75 |

5. EtOAc solvate E: 60 mg of Compound (1) was added to 1 mL of EtOAc. The suspension was cooled to 10 ° C and stirred for 4 days. The sample was then filtered and analyzed for XRPD. Characteristics of EtOAc solvate E: Certain representative XRPD peaks of EtOAc solvate E are summarized in Table 14 below.

**Table 14: Certain representative XRPD of EtOAc solvate E solvate of Compound (1)**

| | EtOAc solvate E | |
|---|---|---|
| **XRPD Peaks** | **Angle (2-Theta ± 0.2)** | **Intensity %** |
| 1 | 9.7 | 57 |
| 2 | 11.6 | 35 |
| 3 | 12.4 | 62 |
| 4 | 15.7 | 36 |
| 5 | 18.8 | 100 |
| 6 | 23.9 | 29 |

6. EtOAc solvate F: Compound (1) (30.46 g, 66.27 mmol) was charged into a 500 ml round bottom flask. Charged 2-Me-THF (182.8 mL) and started agitation. Sodium hydroxide (122.6 mL of 2 M, 245.2 mmol) was then charged to the solution. The reaction mixture was heated to 68 °C and stirred overnight at 70 °C. The reaction was cooled to 0 °C. Citric acid (157.0 mL of 30 %w/v, 245.2 mmol) was added. The resulting mixture was stirred for 30 minutes. Phases were separated and water (152.3 mL) was added to the organic layer. The phases were allowed to separate. The batch was distilled down to 3 volumes. 2-MeTHF (91.38 mL) was added and the batch was distilled down to 3 vol. The batch was distilled down to 3 volumes. 2-MeTHF (91.38 mL) was added and the batch was distilled down to 3 vol EtOAc (304.6 mL) was charged and the batch was distilled down to 2-3 volumes. The batch was adjusted to 10 volumes by adding 7-8 volumes of EtOAc. The batch was distilled down to 2-3 volumes. The batch was adjusted to 10 volumes by adding 7-8 volumes of EtOAc. The batch was distilled down to 2-3 volumes. The batch was adjusted to 10 volumes by adding 7-8 volumes of EtOAc. Adjusted batch volume to 10 volume total and stir heat batch to 50 °C. A small sample was taken and filtered after the temperature of 50° was reached. TGA data indicated an EtOAc solvate with a stoichiometry of approximately 2: 1 (Compound (1):EtOAc). Characteristics of EtOAc solvate F: Certain representative XRPD peaks of EtOAc solvate F are summarized in Table 15 below.

**Table 15: Certain representative XRPD of EtOAc solvate F**

| | EtOAc solvate F |
|---|---|
| **XRPD Peaks** | **Angle (2-Theta ± 0.2)** |
| 1 | 7,2 |
| 2 | 9.7 |
| 3 | 17.1 |
| 4 | 18.8 |
| 5 | 22.7 |
| 6 | 23.5 |

7. EtOAc Solvate G: 1 g of Compound (1) was added to 5 mL of EtOAc. The suspension was stirred at room temperature for 1 day. Alternatively, 100 mg of ethyl acetate solvate seeds were added into the suspension of Compound (1) in EtOAc and the resulting mixture was stirred at room temperature for a day. The sample was then filtered and analyzed for XRPD. TGA data indicated an EtOAc solvate with a stoichiometry of approximately 1: 1 (Compound (1) • EtOAc). Characteristics of EtOAc solvate G: Certain representative XRPD peaks of EtOAc solvate G are summarized in Table 16 below.

**Table 16: Certain representative XRPD of EtOAc solvate G**

| | EtOAc solvate G |
|---|---|
| **XRPD Peaks** | **Angle (2-Theta ± 0.2)** |
| 1 | 7.5 |
| | |
| 2 | 12.1 |
| 3 | 13.0 |
| 4 | 13.7 |
| 5 | 16.2 |
| 6 | 19.7 |

4C: Formation of n-Butylacetate Solvates of Compound (1) (Compound (1) • nBuOAc) n-Butylacetate solvates A-C of Compound (1) (Compound (1) • nBuOAc) can be prepared by following the steps described below:
1. n-Butylacetate Solvate A: A mixture of 500 mg of Compound (1) in 5 mL of n-BuOAc was stirred for 3 days in a capped 20 dram vial. The solids were collected by filtration and analyzed. TGA data (not shown) indicated an n-BuOAc solvate with a stoichiometry of approximately 2: 1 (Compound (1) • n-BuOAc). Characteristics of n-Butylacetate solvate A of Compound (1): Certain representative XRPD peaks of n-Butylacetate solvate A are summarized in Table 17 below.

**Table 17: Certain representative XRPD of n-Butylacetate Solvate A**

| | *n*-Butylacetate Solvate A |
|---|---|
| **XRPD Peaks** | **Angle (2-Theta ± 0.2)** |
| 1 | 9.7 |
| 2 | 14.9 |
| 3 | 16.5 |
| 4 | 19.6 |
| 5 | 20.0 |
| 6 | 21.0 |

2. n-Butylacetate Solvate B: 109 mg of Compound (1) was dissolved in 2 mL of n-BuOAc. Precipitation began to occur after a few minutes. The solvent was then evaporated under ambient conditions for 2 weeks. The resulting material was collected and characterized. TGA data (not shown) indicated an n-BuOAc solvate with a stoichiometry of approximately 1: 1 (Compound (1) • n-BuOAc). Characteristics of n-Butylacetate solvate B of Compound (1): Certain representative XRPD peaks of n-Butylacetate solvate B are summarized in Table 18 below.

**Table 18: Certain representative XRPD of n-Butylacetate Solvate B of Compound (1) n-Butylacetate Solvate B**

| | *n*-Butylacetate Solvate B |
|---|---|
| **XRPD Peaks** | **Angle (2-Theta ± 0.2)** |
| 1 | 6.4 |
| 2 | 6.9 |
| 3 | 17.5 |
| | |
| 4 | 18.2 |
| 5 | 18.9 |
| 6 | 23.2 |

3. n-Butylacetate Solvate C: A mixture of Compound (1) and n-BuOAc was stirred at room temperature similarly as described above for n-Butylacetate solvates A and B. TGA data indicated an n-BuOAc solvate with a stoichiometry of approximately 4: 1 (Compound (1) • n-BuOAc). Characteristics of n-Butylacetate solvate C of Compound (1): Certain representative XRPD peaks of n-Butylacetate Solvate C are summarized in Table 19 below.

**Table 19: Certain representative XRPD of n-Butylacetate Solvate C of Compound (1)**

| | *n*-Butylacetate Solvate C |
|---|---|
| **XRPD Peaks** | **Angle (2-Theta ± 0.2)** |
| 1 | 6.9 |
| 2 | 9.6 |
| 3 | 15.9 |
| 4 | 16.9 |
| 5 | 18.6 |
| 6 | 19.3 |

The invention relates to an administration unit comprising the amorphous form of Compound (1) or any of the polymorphic forms of Compound (1) according to the invention. The administration unit according to the invention is useful for treating various diseases and disorders which include but are not limited to inhibiting or reducing the activity of HCV polymerase in a biological in vitro sample or in a subject, and of treating a HCV infection in a subject.

### SPECIFIC INORMATION CONCERNING ASPECT (II) OF THE INVENTION

Aspect (ii) of the invention concerns solvates of Compound (1) and relates to administration units comprising Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, respectively. The invention relates to a solid form of Compound (1), namely to a solvate of Compound (1), which is useful for treating inhibiting or reducing the activity of HCV polymerase in a biological in vitro sample or in a subject, treating a HCV infection in a subject. Solvates of Compound (1) are described in WO2013016491, which is incorporated by reference.

Compound (1) is represented by

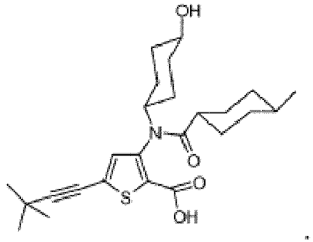

Preferred solvates of Compound (1) are selected from the group consisting of: Compound (1) • H₂O, wherein the Compound (1) • H₂O are in a molar ratio of 1:0.5 to 1:3 (Compound (1) : H₂O), Compound (1) • methanol, Compound (1) • ethanol • isopropanol, Compound (1) • acetone, Compound (1) • ethylacetate, Compound (1) • isopropylacetate, Compound (1) • ethylacetate • 2-methyl THF, and Compound (1) • ethanol (see also WO2013016491).

As used herein, a solvate of Compound (1) is a form of Compound (1) characterized by and can be obtained by as described in WO2013016491 as follows:

The present invention generally relates to solvates of Compound (1), to methods of inhibiting or reducing the activity of HCV polymerase in a biological in vitro sample or in a subject, to methods of treating a HCV infection in a subject, which employ the solvates of Compound (1), and to methods of preparing the solvates of Compound (1):

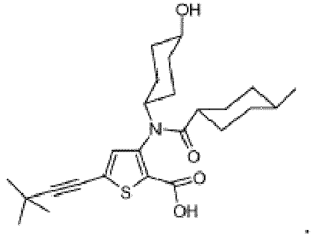

In one embodiment, the present invention is directed to a solvate of Compound (1) selected from the group consisting of: Compound (1) • H₂O, wherein the Compound (1) • H₂O are in a molar ratio of 1:0.5 to 1:3 (Compound (1) • H₂O), Compound (1) • methanol, Compound (1) • ethanol • isopropanol, Compound (1) • acetone, Compound (1) • ethylacetate, Compound (1) • isopropylacetate, Compound (1) • ethylacetate • 2-methyl THF, and Compound (1) • ethanol. In another embodiment, the present invention is directed to a pharmaceutical composition comprising a solvate of Compound (1) • described herein and at least one pharmaceutically acceptable carrier or excipient. In yet another embodiment, the present invention is directed to a method of inhibiting or reducing the activity of HCV polymerase in a biological in vitro sample. The method includes administering to the sample an effective amount of a solvate of Compound (1) described herein.

In yet another embodiment, the present invention is directed to a method of inhibiting or reducing the activity of HCV polymerase in a subject. The method includes administering to the subject an effective amount of a solvate of Compound (1) described herein. In yet another embodiment, the present invention is directed to a method of treating a HCV infection in a subject. The method includes administering to the subject an effective amount of a solvate of Compound (1) described herein. Methods of preparing solvates of Compound (1) described herein are also provided. The methods employ stirring Compound (1) in a suitable solvent to form the desired solvate of Compound (1). In one specific embodiment, Compound (1) • H₂O is prepared by stirring a mixture of Compound (1) and 1 H₂O at room temperature. In another specific embodiment, Compound (1) • methanol is prepared by stirring a mixture of Compound (1) and methanol at room temperature. In yet another specific embodiment, Compound (1) • ethanol • isopropanol is prepared by stirring a mixture of Compound (1) and a mixture of ethanol and isopropanol at room temperature. In yet another specific embodiment, Compound (1) • acetone is prepared by stirring a mixture of Compound (1) and acetone at room temperature. In yet another embodiment, Compound (1) • ethylacetate is prepared by stirring a mixture of Compound (1) and ethylacetate at a temperature in a range of 5 °C to 35 °C. In yet another specific embodiment, Compound (1) • isopropylacetate is prepared by stirring a mixture of Compound (1) and isopropylacetate at room temperature. In yet another embodiment, Compound (1) • ethylacetate • 2-methyl THF is prepared by stirring a mixture of Compound (1), ethylacetate and 2-methyl THF at a temperature in a range of 5 °C to 35 °C. In yet another specific embodiment, Compound (1) ethanol is prepared by stirring a mixture of Compound (1) and ethanol at room temperature. In yet another specific embodiment, Compound (1) • n-butylacetate is prepared by stirring a mixture of Compound (1) and n-butylacetate at room temperature. In yet another specific embodiment, Compound (1) • heptane is prepared by stirring a mixture of Compound (1) and heptane at room temperature. In yet another specific embodiment, Compound (1) • 2-butanone is prepared by stirring a mixture of Compound (1) and 2-butanone at room temperature. In yet another specific embodiment, Compound (1) • methylacetate is prepared by stirring a mixture of Compound (1) and methylacetate at room temperature. Use of the solvates of Compound (1) described herein for inhibiting or reducing the activity of HCV polymerase in a biological in vitro sample or in a subject is also provided. Uses of the solvates of Compound (1) described herein for treating a HCV infection in a subject are also provided. The present invention also provides use of the solvates of Compound (1) described herein for the manufacture of a medicament for treating a HCV infection in a subject. FIGs. 1-23 of WO2013016491 show room temperature XRPD patterns of certain solvates of Compound (1): FIG. 1 of WO2013016491: Compound (1) • H₂O, wherein the Compound (1) • H₂O are in a molar ratio of 1: 1 (Compound (1) : H₂O) (hydrate A); FIG. 2 of WO2013016491: Compound (1) • H₂O, wherein the Compound (1) • H₂O are in a molar ratio of 1:2 (Compound (1) : H₂O) (hydrate B); FIG. 3 of WO2013016491: Compound (1) • methanol; FIG. 4 of WO2013016491: Compound (1) • ethanol • isopropanol; FIG. 5 of WO2013016491: Compound (1) • acetone; FIG. 6 of WO2013016491: Compound (1) • ethylacetate (ethylacetate solvate A); FIG. 7 of WO2013016491: Compound (1) • ethylacetate (ethylacetate solvate B); FIG. 8 of WO2013016491: Compound (1) • ethylacetate (ethylacetate solvate C); FIG. 9 of WO2013016491: Compound (1) • ethylacetate (ethylacetate solvate D); FIG. 10 of WO2013016491: Compound (1) • ethylacetate (ethylacetate solvate E); FIG. 11 of WO2013016491: Compound (1) • ethylacetate (ethylacetate solvate F); FIG. 12 of WO2013016491: Compound (1) • ethylacetate (ethylacetate solvate G); FIG. 13 of WO2013016491: Compound (1) • ethylacetate • 2-methyl THF (ethylacetate/2-methyl THF:70%/30% w/w); FIG. 14 of WO2013016491: Compound (1) • ethylacetate • 2-methyl THF (ethylacetate/2-methyl THF: 90%/10% w/w); FIG. 15 of WO2013016491: Compound (1) • ethanol; FIG. 16 of WO2013016491: Compound (1) • n-butylacetate (n-butylacetate Solvate A); FIG. 17 of WO2013016491: Compound (1) • n-butylacetate (n-butylacetate Solvate A); FIG. 18 of WO2013016491: Compound (1) • n-butylacetate (n-butylacetate Solvate C); FIG. 19 of WO2013016491: Compound (1) • heptane (heptane Solvate A); FIG. 20 of WO2013016491: Compound (1) • heptane (heptane Solvate B); FIG. 21 of WO2013016491: Compound (1) • heptane (heptane Solvate C); FIG. 22 of WO2013016491: Compound (1) • heptane (heptane Solvate D); and FIG. 23 of WO2013016491: Compound (1) • heptane (heptane Solvate E).FIGs. 24 and 25 of WO2013016491 show solid state C¹³ NMR spectra of certain solvates of Compound (1): FIG. 24: of WO2013016491 Compound (1) • 2-butanone (methylethylketone (MEK)); and FIG. 25 of WO2013016491: Compound (1) • methylacetate.

Compound (1) represented by the following structural formula: 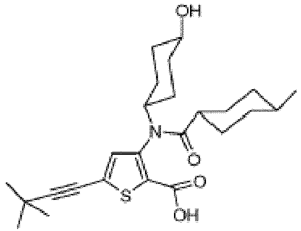 and pharmaceutically acceptable salts thereof are a NS5B polymerase inhibitors, and also described in WO 2008/058393.

The term "solvate" as used herein is a solvate formed from the association of one or more solvent molecules (e.g., H₂O, acetone, etc.) to Compound (1). The solvates of Compound (1) can include a stoichiometric or non-stoichiometric amount of solvent bound by non-covalent intermolecular forces.

In a preferred embodiment, the present invention is directed to methods of preparing solvates of Compound (1). In general, solvates of Compound (1) can be prepared by stirring a mixture of Compound (1) and a desired solvent at a suitable temperature (e.g., room temperature (20 °C -28 °C), 5 °C - 50 °C, 10°C - 50 °C, 10 °C - 35 °C, or 20 °C - 25 °C) for a sufficient time to form the desired solvates of Compound (1). For example, the desired solvent is 1 H₂O for Compound (1) • H₂O; methanol for Compound (1) • methanol; a mixture of ethanol and isopropanol for Compound (1) • ethanol • isopropanol; acetone for Compound (1) • acetone; ethylacetate for Compound (1) • ethylacetate; isopropylacetate for Compound (1) • isopropylacetate; a mixture of ethylacetate and 2-methyl THF for Compound (1) • ethylacetate • 2-methyl THF; and ethanol for Compound (1) • ethanol. In one specific embodiment, the hydrates of Compound (1) can be prepared by stirring a mixture of Compound (1) and 1 H₂O at room temperature. In another specific embodiment, the methanol solvates of Compound (1) can be prepared by stirring a mixture of Compound (1) and methanol at room temperature. In yet another specific embodiment, the ethanol/isopropanol solvates of Compound (1) can be prepared by stirring a mixture of Compound (1) a mixture of ethanol and isopropanol at room temperature. In yet another specific embodiment, the acetone solvates of Compound (1) can be prepared by stirring a mixture of Compound (1) and acetone at room temperature. In yet another specific embodiment, the ethylacetate solvates of Compound (1) can be prepared by stirring a mixture of Compound (1) and ethylacetate at a temperature in a range of 5 °C to 50 °C, 10 °C to 50 °C, or 5 °C to 35 °C. In yet another specific embodiment, the isopropylacetate solvates of Compound (1) can be prepared by stirring a mixture of Compound (1) and isopropylacetate at room temperature. In yet another specific embodiment, the EtOAc/2-methyl THF solvates of Compound (1) can be prepared by stirring a mixture of Compound (1), EtOAc, 2-methyl THF at a temperature in a range of 5 °C to 35 °C, such as at 5 °C or at room temperature. In yet another specific embodiment, the ethanol solvates of Compound (1) can be prepared by stirring a mixture of Compound (1) and ethanol at room temperature. In yet another specific embodiment, the n-butylacetate, heptane, 2-butanone, methylacetate solvates of Compound (1) can each and independently be prepared by stirring a mixture of Compound (1) and the respective solvent: n-butylacetate for the n-butylacetate solvates; heptane for the heptane solvate; 2-butanone (MEK) for the 2-butanone solvates; methylacetate for the methylacetate solvates, at room temperature.

The present invention encompasses solvates of Compound (1) described above in isolated, pure form, or in a mixture as a solid composition when admixed with other materials, for example the other known polymorphic forms (i.e. amorphous form, Form A of Compound (1), or other forms) or solvates of Compound (I), or any other materials. Thus in one aspect there are provided solvates of Compound (1) in isolated solid form. In a further aspect there are provided solvates of Compound (1) in pure form. The pure form means that a certain solvate of Compound (1) is over 95% (w/w), for example, over 98%

(w/w), over 99% (w/w %), over 99.5% (w/w), or over 99.9% (w/w). Assaying the solid phase for the presence of the crystalline solvates of Compound (1) and the co-crystal former may be carried out by conventional methods known in the art. For example, powder X-ray diffraction techniques can be used to assess the presence of crystalline solvates of Compound (1). Other techniques, used in an analogous fashion, include differential scanning calorimetry (DSC), thermogravimetric analysis (TGA), solid state NMR spectroscopy, Raman spectroscopy, and single crystal X-ray diffraction.

The experimental section of WO2013016491 contains the folloiwng information:

### Compound (1):

### A: Recrystallization in a mixture of nBuOAc and acetone:

A solvent switch from 2-Me-THF to nBuOAc was performed by first reducing the batch volume to 2-3 volumes (based on compound (D) charge) by vacuum distillation at a maximum temperature of 45 °C. nBuOAc (3 vol, based on compound (D) charge) was added and the batch volume was reduced to 2-3 volumes (based on compound (D) charge) via vacuum distillation at a maximum temperature of 45 °C. The batch volume was then adjusted to a total of 5-6 volumes by addition of nBuOAc. The solution was analyzed for residual 2-Me-THF in content in nBuOAc. This cycle was repeated until less than 1% of 2-Me-THF with respect to nBuOAc remained, as determined by GC analysis. Once the residual 2-Me-THF IPC criterion was met and it was insured that the total batch volume is 6 (based on compound (D) charge), the batch temperature was adjusted to 40 - 45 °C. Acetone is then charged into the batch to have approximately 10 wt% acetone in the solvent. The batch temperature was adjusted to 40 - 45 °C. Compound (1) seed (1.0% by weight with respect to the total target weight of Compound (1)) was added. The batch was agitated at 40 - 45 °C for 4-8 hours. The recrystallization progress is monitored by X-ray powder diffraction (XRPD). If spectrogram matched that of required form, then the batch was cooled from 40 - 45 °C to 30-35 °C (preferably about 35°C) at rate of 5 °C/hour. The batch was held at about 35°C for at least one hour, and then filtered and the filter cake was washed with 9:1 w wt mixture of nBuOAc/acetone (1 vol). The material was dried in vacuum with nitrogen bleed at NMT 45 °C for 12 - 24 hours. The expected isolated molar yield of Compound (1) (Form M) starting with compound (D) was 80-85%. Compound (1): 1H NMR (400 MHz, DMSO-<¾) 0.58 (m, 1H), 0.74 (q, J = 6.53 Hz, 1H), 0.81 (ddd, J = 12.86, 12.49, 3.19 Hz, 1H), 1.18 (m, 5H), 1.28 (s, 3H), 1.42 (m, 1H), 1.55 (m, 3H), 1.61 (m, 1H), 1.73 (m, 2H), 1.81 (m, 2H), 3.19 (m, 1H), 4.26 (m, 1H), 4.49 (bs, 1H), 7.14 (s, 1H), 13.45 (bs, 1H).

### B: Recrystallization in EtOAc:

A solvent switch from 2-Me-THF to EtOAc was performed by first reducing the batch volume to 2-3 volumes (based on compound (D) charge) by vacuum distillation at a maximum temperature of 35 °C. EtOAc (10 vol, based on compound (D) charge) was added and the batch volume was reduced to 2-3 volumes (based on compound (D) charge) via vacuum distillation at a maximum temperature of 35 °C. The solution was analyzed for residual 2-Me-THF in content in EtOAc. This cycle was repeated until less than 1% of Me-THF with respect to EtOAc remained, as determined by GC analysis. Once the residual 2-Me-THF IPC criterion was met and it was insured that the total batch volume is 10 (based on compound (D) charge), the batch temperature was adjusted to 40 - 45 °C. Compound (1) seed (1.0% by weight with respect to the total target weight of Compound (1)) was added. The batch was agitated at 40 - 45 °C for 12 hours. A flat floor / flat bottomed reactor (not conical) should be used. The recrystallization progress is monitored by X-ray powder diffraction (XRPD). If spectrogram matched that of required form, then the batch was cooled from 40 - 45 °C to 11 - 14 °C at rate of 5 °C/hour. The batch was filtered and the filter cake was washed with EtOAc (1 vol), previously chilled to 11 - 14 °C. The material was dried in vacuum with nitrogen bleed at NMT 45 °C for 12 - 24 hours. The expected isolated molar yield of Compound (1) (Form M) starting with compound (D) was 80-85%.

Example 3: Formation of Solvates of Compound (1)

3A: Formation of Hydrate A (Compound (1) • 1H₂O): Hydrate A of Compound (1) can be prepared by following the steps described below: 121 mg of Compound (1) was charged to a vial. 2 mL of DI water was then charged to the vial. The mixture was stirred at room temperature for 2 days to form Compound (1) • 1 H₂O and the resulting solids were filtered and dried. TGA data indicated a hydrate solvate with a stoichiometry of approximately 1: 1 (Compound (1) • H₂O). Characteristics of Hydrate A of Compound (1): XRPD data of hydrate A of Compound (1) is shown in FIG. 1 of WO2013016491. Certain representative XRPD peaks of hydrate A of Compound (1) are summarized in Table 1 below.

**Table 1: Certain representative XRPD Peaks of Hydrate A of Compound (1)**

| | **Hydrate A** | |
|---|---|---|
| **XRPD Peaks** | **Angle (2-Theta ± 0.2)** | **Intensity %** |
| 1 | 20.3 | 100.0 |
| 2 | 9.1 | 41.1 |
| 3 | 18.4 | 31.5 |
| 4 | 7.6 | 27.3 |
| 5 | 19.6 | 25.7 |
| 6 | 14.6 | 24.4 |

3B: Formation of Hydrate B (Compound (1) • 2H₂O): Hydrate B of Compound (1) can be prepared by following the steps described below: 20 mg of Compound (1) was charged to a vial. 0.5 mL of DI water was then charged to the vial. The mixture was stirred at room temperature for 3 weeks to form Compound (1) • 2 H₂O and the resulting solids were filtered and dried. TGA data indicated a hydrate solvate with a stoichiometry of approximately 1:2 (Compound (1) • H₂O). Characteristics of Hydrate B of Compound (1): XRPD data of hydrate B of Compound (1) is shown in FIG. 2 of WO2013016491. Certain representative XRPD peaks and DSC endotherm (°C) of hydrate B of Compound (1) are summarized in Table 2 below.

Certain representative XRPD Peaks and DSC Endotherm of Hydrate B of Compound

| | **Hydrate B** | |
|---|---|---|
| **DSC Endotherm (°C)** | 60 °C | |
| | | |

| **XRPD Peaks** | **Angle (2-Theta ± 0.2)** | **Intensity %** |
|---|---|---|
| 1 | 19.0 | 100.0 |
| 2 | 9.9 | 84.2 |
| 3 | 12.9 | 36.5 |
| 4 | 11.6 | 31.9 |
| 5 | 15.9 | 27.6 |
| 6 | 24.2 | 20.3 |

3C: Formation of Methanol Solvates of Compound (1) (Compound (1) • MeOH): Methanol solvates of Compound (1) can be prepared by following the steps described below: A slurry 20 mg of Compound (1) in 500 microliters of MeOH was stirred at room temperature for 3 weeks in a capped HPLC vial to form Compound (1) • MeOH. The solids were collected by filtration and analyzed by XRPD. TGA data indicated a methanol solvate with a stoichiometry of approximately 1: 1 (Compound (1) • methanol). Characteristics of methanol solvate A of Compound (1): XRPD data of methanol solvate A of Compound (1) is shown in FIG. 3 of WO2013016491. Certain representative XRPD peaks of methanol solvate A of Compound (1) are summarized in Table 3 below.

**Table 3: Certain representative XRPD of Methanol Solvate A of Compound (1)**

| | **Methanol Solvate A** | |
|---|---|---|
| **XRPD Peaks** | **Angle (2-Theta ± 0.2)** | **Intensity %** |
| 1 | 8.0 | 100 |
| 2 | 10.3 | 68 |
| 3 | 17.9 | 59 |
| 4 | 19.9 | 63 |
| 5 | 20.6 | 39 |
| 6 | 22.1 | 45 |

3D: Formation of Ethanol/Isopropanol Solvates of Compound (1) (Compound (1) • EtOH • IPA): Ethanol/Isopropanol solvates of Compound (1) (94.7 vol% EtOH/ 5.3 vol% IPA) can be prepared by following the steps described below: A slurry containing 100 mg of Compound (1) in EtOH/IPA (95.7% EtOH / 4.7% IPA) in a 2 mL vial was stirred at room temperature overnight to form Compound (1) • EtOH • IPA. The solvent was decanted off, giving the remaining wet-cake which was analyzed by XRPD. Characteristics of EtOH/IPA solvates of Compound (1): XRPD data of EtOH/IPA solvates of Compound (1) is shown in FIG. 4 of WO2013016491. Certain representative XRPD peaks of EtOH/IPA solvates of Compound (1) are summarized in Table 4 below.

**Table 4: Certain representative XRPD of EtOH/IPA Solvates of Compound (1)**

| | **EtOH/IPA Solvates** | |
|---|---|---|
| **XRPD Peaks** | **Angle (2-Theta ± 0.2)** | **Intensity %** |
| 1 | 8.7 | 18 |
| 2 | 9.3 | 77 |
| 3 | 18.3 | 100 |
| 4 | 19.8 | 24 |
| 5 | 15.5 | 45 |
| 6 | 23.1 | 18 |

3E: Formation of Acetone Solvates of Compound (1) (Compound (1) • acetone): Acetone solvates of Compound (1) (Compound (1) • 1 acetone) can be prepared by following the steps described below: Crystals of acetone solvate of Compound (1) (1: 1 stoichiometry) were grown by slow evaporation from a solution of Compound (1) in acetone. The crystals were collected and analyzed by XRPD. TGA data indicated an acetone solvate with a stoichiometry of approximately 1: 1 (Compound (1) • acetone). Characteristics of acetone solvates of Compound (1): XRPD data of acetone solvates of Compound (1) is shown in FIG. 5 of WO2013016491. Certain representative XRPD peaks of acetone solvates of Compound (1) are summarized in Table 5 below.

**Table 5: Certain representative XRPD of Acetone Solvates of Compound (1)**

| | Acetone Solvates | |
|---|---|---|
| **XRPD Peaks** | **Angle (2-Theta ± 0.2)** | **Intensity %** |
| 1 | 7.7 | 100 |
| 2 | 10.4 | 63 |
| 3 | 11.3 | 43 |
| 4 | 16.5 | 69 |
| 5 | 19.1 | 37 |
| 6 | 21.1 | 83 |

3F: Formation of EthylAcetate Solvates of Compound (1) (Compound (1) • EtOAc): EtOAc solvates A-F of Compound (1) (Compound (1) • EtOAc) can be prepared by following the steps described below:
1. EtOAc solvate A: A slurry containing 100 mg of Compound (1) in EtOAc in a 2 mL vial was stirred at room temperature overnight. The solvent was decanted off giving the remaining wet-cake which was analyzed by XRPD. TGA data indicated an EtOAc solvate with a stoichiometry of approximately 3: 1 (Compound (1) • EtOAc). Characteristics of EtOAc solvate A: XRPD data of EtOAc solvate A is shown in FIG. 6 of WO2013016491. Certain representative XRPD peaks of EtOAc solvate A are summarized in Table 6 below.

**Table 6: Certain representative XRPD of EtOAc solvate A**

| | EtOAc solvate A | |
|---|---|---|
| **XRPD Peaks** | **Angle (2-Theta 0.2)** | **Intensity %** |
| 1 | 6.4 | 96 |
| 2 | 7.0 | 100 |
| 3 | 9.7 | 62 |
| 4 | 16.1 | 49 |
| 5 | 18.7 | 59 |
| 6 | 22.6 | 47 |

2. EtOAc solvate B: A slurry containing 20 mg of Compound (1) in 500 microliters of EtOAc in a capped vial was stirred at room temperature for 3 weeks. The solids were collected by filtration and analyzed by XRPD. Characteristics of EtOAc solvate B: XRPD data of EtOAc solvate B is shown in FIG. 7 of WO2013016491. Certain representative XRPD peaks of EtOAc solvate B are summarized in Table 7 below.

**Table 7: Certain representative XRPD of EtOAc solvate B**

| | EtOAc solvate B |
|---|---|
| **XRPD Peaks** | **Angle (2-Theta ± 0.2)** |
| 1 | 6.4 |
| 2 | 7.3 |
| 3 | 8-1 |
| 4 | 9.0 |
| 5 | 18.1 |
| 6 | 19.7 |

3. EtOAc solvate C: Approximately 20 kg of Compound (1) was added to a reactor. 200 kg of 2-MeTHF was then charged to the reactor. 200 kg of EtOAc was then added to the reactor and the solution was rotovapped at 100 mmHg and 30°C which resulted in an oil being obtained. The reactor was then charged with 591 kg of EtOAc which was then rotovapped at 50 mmHg and 30°C. The solid residue was submitted for XRPD. Characteristics of EtOAc solvate C: XRPD data of EtOAc solvate C is shown in FIG. 8 of WO2013016491. Certain representative XRPD peaks of EtOAc solvate C are summarized in Table 8 below.

**Table 8: Certain representative XRPD of EtOAc solvate C**

| | EtOAc solvate C | |
|---|---|---|
| **XRPD Peaks** | **Angle (2-Theta ± 0.2)** | **Intensity %** |
| 1 | 6.3 | 100 |
| 2 | 14.6 | 17 |
| 3 | 15.6 | 13 |
| 4 | 18.9 | 34 |
| 5 | 20.4 | 15 |
| 6 | 22.2 | 25 |

4. EtOAc solvate D: 550 mg of Compound (1) was added to 2 mL of EtOAc. The slurry was shaken for 4 days at 400 rpm between 20°C and 25°C. The sample was then filtered and analyzed for XRPD. Characteristics of EtOAc solvate D: XRPD data of EtOAc solvate D is shown in FIG. 9 of WO2013016491. Certain representative XRPD peaks of EtOAc solvate D are summarized in Table 9 below.

**Table 9: Certain representative XRPD of EtOAc solvate D**

| | EtOAc solvate D | |
|---|---|---|
| **XRPD Peaks** | **Angle (2-Theta ± 0.2)** | **Intensity %** |
| 1 | 4.9 | 43 |
| 2 | 9.8 | 71 |
| 3 | 14.6 | 48 |
| 4 | 16.6 | 100 |
| 5 | 20.2 | 91 |
| 6 | 21.2 | 75 |

5. EtOAc solvate E: 60 mg of Compound (1) was added to 1 mL of EtOAc. The suspension was cooled to 10° C stirred for 4 days. The sample was then filtered and analyzed for XRPD. Characteristics of EtOAc solvate E: XRPD data of EtOAc solvate E is shown in FIG. 10 of WO2013016491. Certain representative XRPD peaks of EtOAc solvate E are summarized in Table 10 below.

**Table 10: Certain representative XRPD of EtOAc solvate E solvate of Compound (1)**

| | EtOAc solvate E | |
|---|---|---|
| **XRPD Peaks** | **Angle (2-Theta ± 0.2)** | **Intensity %** |
| 1 | 9.7 | 57 |
| 2 | 11.6 | 35 |
| | | |
| 3 | 12.4 | 62 |
| 4 | 15.7 | 36 |
| 5 | 18.8 | 100 |
| 6 | 23.9 | 29 |

6. EtOAc solvate F: Compound (1) (30.46 g, 66.27 mmol) was charged o a 500 ml round bottom flask Charged 2-Me-THF (182.8 mL) and started agitation. Sodium hydroxide (122.6 mL of 2 M, 245.2 mmol) was then charged to the solution. The reaction mixture was heated to 68 °C and stirred overnight at 70 °C. The reaction was cooled to 0 °C. Citric acid (157.0 mL of 30 %w/v, 245.2 mmol) was added. The resulting mixture was stirred for 30 minutes. Phases were separated and water (152.3 mL) was added to the organic layer. The phases were allowed to separate. The batch was distilled down to 3 volume. 2-MeTHF (91.38 mL) was added and the batch was distilled down to 3 vol. The batch was distilled down to 3 volume. 2-MeTHF (91.38 mL) was added and the batch was distilled down to 3 vol EtOAc (304.6 mL) was charged and the batch was distilled down to 2-3 volumes. The batch was adjusted to 10 volumes by adding 7-8 volumes of EtOAc. The batch was distilled down to 2-3 volumes. The batch was adjusted to 10 volumes by adding 7-8 volumes of EtOAc. The batch was distilled down to 2-3 volumes. The batch was adjusted to 10 volumes by adding 7-8 volumes of EtOAc. Adjusted batch volume to 10 volume total and stir heat batch to 50 °C. A small sample was taken and filtered after the temperature of 50° was reached. TGA data indicated an EtOAc solvate with a stoichiometry of approximately 2: 1 (Compound (1) • EtOAc). Characteristics of EtOAc solvate F: XRPD data of EtOAc solvate F is shown in FIG. 11 of WO2013016491. Certain representative XRPD peaks of EtOAc solvate F are summarized in Table 11 below.

**Table 11: Certain representative XRPD of EtOAc solvate F**

| | EtOAc solvate F |
|---|---|
| **XRPD Peaks** | **Angle (2-Theta ± 0.2)** |
| 1 | 7.2 |
| 2 | 9.7 |
| 3 | 17.1 |
| 4 | 18.8 |
| 5 | 22.7 |
| 6 | 23.5 |

7. EtOAc Solvate G: 1 g of Compound (1) was added to 5 mL of EtOAc. The suspension was stirred at room temperature for 1 day. Alternatively, 100 mg of ethylacetate solvate seeds were added into the suspension of Compound (1) in EtOAc and the resulting mixture was stirred at room temperature for a day. The sample was then filtered and analyzed for XRPD. TGA data indicated an EtOAc solvate with a stoichiometry of approximately 1: 1 (Compound (1) • EtOAc). Characteristics of EtOAc solvate G: XRPD data of EtOAc solvate G is shown in FIG. 12 of WO2013016491. Certain representative XRPD peaks of EtOAc solvate G are summarized in Table 12 below.

**Table 12: Certain representative XRPD of EtOAc solvate G**

| | EtOAc solvate G |
|---|---|
| **XRPD Peaks** | **Angle [2-Theta ± 0.2]** |
| 1 | 7.5 |
| 2 | 12.1 |
| 3 | 13.0 |
| 4 | 13.7 |
| 5 | 16.2 |
| 6 | 19.7 |

3G: Formation of Isopropyl Acetate Solvates of Compound (1) (Compound (1) • IPac): A slurry containing 100 mg of Compound (1) in isopropylacetate in a 2 mL vial was stirred at room temperature overnight. The solvent was decanted off giving the remaining wet-cake which was analyzed by XRPD.

Characteristics of isopropylacetate solvate of Compound (1): XRPD data of isopropylacetate solvate of Compound (1) showed that the ethylacetate solvate A of Compound (1) and isopropylacetate solvate of Compound (1) were isostructural to each other, sharing the same representative XRPD peaks summarized in Table 13 below.

**Table 13: Certain representative XRPD of isopropyl acetate solvate of Compound (1)**

| | Isopropylacetate Solvate | |
|---|---|---|
| **XRPD Peaks** | **Angle (2-Theta ± 0.2)** | **Intensity %** |
| 1 | 6.4 | 96 |
| 2 | 7.0 | 100 |
| 3 | 9.7 | 62 |
| 4 | 16.1 | 49 |
| 5 | 18.7 | 59 |
| 6 | 22.6 | 47 |

3H: Formation of Ethylacetate/ 2-Methyl THF Solvates of Compound (1) (Compound (1) • ethylacetate • 2-methyl THF):
1. Solvate A: EtOAc / 2-MethylTHF (70%/30% w/w): A slurry 100 mg of Compound (1) in 1 mL of 70% EtOAc / 30% 2-MethylTHF (w/w) at 5°C was stirred for 24 hours in a capped vial. The solids were collected by filtration and analyzed by XRPD. Characteristics of ethylacetate/ 2-methyl THF solvates of Compound (1): XRPD data is shown in FIG. 13 of WO2013016491. Certain representative XRPD peaks are summarized in Table 14 below.

**Table 14: Certain representative XRPD of EtOAc/ 2-methyl THF solvate of Compound (1) (Solvate A)**

| | EtOAc/2-methyl THF Solvate |
|---|---|
| **XRPD Peaks** | **Angle (2-Theta ± 0.2)** |
| 1 | 5.5 |
| 2 | 9.3 |
| 3 | 10.9 |
| 4 | 12.6 |
| 5 | 17.7 |
| 6 | 19.7 |

2. Solvate B: EtOAc / 2-MethylTHF (90%/10% w/w): A slurry 100 mg of Compound (1) in 1 mL of 90% EtOAc / 10% 2-MethylTHF (w/w) at room temperature was stirred for 24 hours in a capped vial. The solids were collected by filtration and analyzed by XRPD. Characteristics of ethylacetate/ 2-methyl THF solvates of Compound (1): XRPD data is shown in FIG. 14 of WO2013016491. Certain representative XRPD peaks are summarized in Table 15 below.

**Table 15: Certain representative XRPD of EtOAc/ 2-methyl THF solvate of Compound (1) (Solvate B)**

| | EtOAc/2-methyl THF Solvate |
|---|---|
| **XRPD Peaks** | **Angle (2-Theta ± 0.2)** |
| 1 | 6.5 |
| 2 | 7.3 |
| 3 | 9.1 |
| 4 | 17.0 |
| 5 | 18.7 |
| 6 | 23.5 |

3I: Formation of Ethanol Solvates of Compound (1) (Compound (1) • Ethanol): A slurry 100 mg of Compound (1) in 500 microliters of EtOH for 24 hours in a capped vial. The solids are collected by filtration and analyzed by XRPD. TGA data indicated an ethanol solvate with a stoichiometry of approximately 1: 1 (Compound (1) • EtOH). Characteristics of ethanol solvates of Compound (1): XRPD data is shown in FIG. 15 of WO2013016491. Certain representative XRPD peaks are summarized in Table 16 below.

**Table 16: Certain representative XRPD of ethanol solvates of Compound (1)**

| | Ethanol Solvate |
|---|---|
| **XRPD Peaks** | **Angle (2-Theta ± 0.2)** |
| 1 | 8.1 |
| 2 | 10.1 |
| 3 | 18.1 |
| 4 | 19.6 |
| 5 | 21.7 |
| 6 | 25.6 |

3J: Formation of n-Butylacetate Solvates of Compound (1) (Compound (1) • nBuOAc): n-Butylacetate solvates A-C of Compound (1) (Compound (1) • nBuOAc) can be prepared by following the steps described below:
1. n-Butylacetate Solvate A: A mixture of 500 mg of Compound (1) in 5 mL of n-BuOAc was stirred for 3 days in a capped 20 dram vial. The solids were collected by filtration and analyzed. TGA data (not shown) indicated an n-BuOAc solvate with a stoichiometry of approximately 2: 1 (Compound (1) • n-BuOAc). Characteristics of n-Butylacetate solvate A of Compound (1): XRPD data of n-Butylacetate solvate A solvate of Compound (1) is shown in FIG. 16 of WO2013016491. Certain representative XRPD peaks of n-Butylacetate solvate A are summarized in Table 17 below.

**Table 17: Certain representative XRPD of n-Butylacetate Solvate A**

| | *n*-Butylacetate Solvate A |
|---|---|
| **XRPD Peaks** | **Angle (2-Theta ± 0.2)** |
| 1 | 9.7 |
| 2 | 14.9 |
| 3 | 16.5 |
| 4 | 19.6 |
| 5 | 20.0 |
| 6 | 21.0 |

2. n-Butylacetate Solvate B: 109 mg of Compound (1) was dissolved in 2 mL of n-BuOAc. Precipitation began to occur after a few minutes. The solvent was then evaporated under ambient conditions for 2 weeks. The resulting material was collected and characterized. TGA data (not shown) indicated an n-BuOAc solvate with a stoichiometry of approximately 1: 1 (Compound (1) • n-BuOAc). Characteristics of n-Butylacetate solvate B of Compound (1): XRPD data of n-Butylacetate solvate B is shown in FIG. 17 of WO2013016491. Certain representative XRPD peaks of n-Butylacetate solvate B are summarized in Table 18 below.

**Table 18: Certain representative XRPD of n-Butylacetate Solvate B of Compound (1)**

| | *n*-Butylacetate Solvate B |
|---|---|
| **XRPD Peaks** | **Angle (2-Theta ± 0.2)** |
| 1 | 6.4 |
| 2 | 6.9 |
| 3 | 17.5 |
| 4 | 18.2 |
| 5 | 18.9 |
| 6 | 23.2 |

3. n-Butylacetate Solvate C: A mixture of Compound (1) and n-BuOAc was stirred at room temperature similarly as described above for n-Butylacetate solvates A and B. TGA data indicated an n-BuOAc solvate with a stoichiometry of approximately 4: 1 (Compound (1) • n-BuOAc). Characteristics of n-Butylacetate solvate C of Compound (1): XRPD data of n-Butylacetate solvate C is shown in FIG. 18 of WO2013016491. Certain representative XRPD peaks of n-Butylacetate Solvate C are summarized in Table 19 below.

**Table 19: Certain representative XRPD of n-Butylacetate Solvate C of Compound (1)**

| | *n*-Butylacetate Solvate C |
|---|---|
| **XRPD Peaks** | **Angle (2-Theta ± 0.2)** |
| 1 | 6.9 |
| 2 | 9.6 |
| 3 | 15.9 |
| 4 | 16.9 |
| 5 | 18.6 |
| 6 | 19.3 |

3K: Formation of Heptane Solvates of Compound (1) (Compound (1) • Heptane): Heptane solvates A-D of Compound (1) (Compound (1) • Heptane can be prepared by following the steps described below:
1. Heptane Solvate A: A mixture of Compound (1) in heptane was stirred at room temperature. The solids obtained after evaporation of heptane at room temperature were collected by filtration and analyzed. Characteristics of heptane solvate A of Compound (1): XRPD data of heptane solvate A is shown in FIG. 19 of WO2013016491. Certain representative XRPD peaks of heptane solvate A are summarized in Table 20 below.

**Table 20: Certain representative XRPD of Heptane Solvate A**

| | Heptane Solvate A |
|---|---|
| **XRPD Peaks** | **Angle (2-Theta ± 0.2)** |
| | |
| 1 | 10.7 |
| 2 | 12.3 |
| 3 | 14.0 |
| 4 | 17.2 |
| 5 | 19.6 |
| 6 | 21.3 |

2. Heptane Solvate B: 106 mg of amorphous Compound (1) was added to a solvent mixture of 0.5 mL EtOAc and 0.5 mL heptane. The suspension was agitated for 7 days at 20°C. The solids were isolated by centrifugation filtration and analyzed. Characteristics of heptane solvate B of Compound (1): XRPD data of heptane solvate B is shown in FIG. 20 of WO2013016491. Certain representative XRPD peaks of heptane solvate B are summarized in Table 21 below.

**Table 21: Certain representative XRPD of Heptane Solvate B**

| | Heptane Solvate B |
|---|---|
| **XRPD Peaks** | **Angle (2-Theta ± 0.2)** |
| 1 | 5.5 |
| 2 | 7.5 |
| 3 | 9.3 |
| 4 | 10.9 |
| 5 | 16.5 |
| 6 | 22.1 |

3. Heptane Solvate C: Slurry in 1 mL of heptane was made by addition of approximately 50 mg of Compound (1). The material was stirred for 60 days at 20°C. The material was then filtered and analyzed by XRPD. Characteristics of heptane solvate C of Compound (1): XRPD data of heptane solvate C is shown in FIG. 21 of WO2013016491. Certain representative XRPD peaks of heptane solvate C are summarized in Table 22 below.

**Table 22: Certain representative XRPD of Heptane Solvate C**

| | Heptane Solvate C |
|---|---|
| **XRPD Peaks** | **Angle (2-Theta ± 0.2)** |
| 1 | 10.4 |
| 2 | 11.2 |
| 3 | 13.4 |
| 4 | 16.9 |
| 5 | 19.3 |
| 6 | 19.8 |

4. Heptane Solvate D: Slurry in 1 mL of heptane was made by addition of approximately 50 mg of Compound (1). The material was stirred for 60 days at 25°C. The material was then filtered and analyzed by XRPD. Characteristics of heptane solvate D of Compound (1): XRPD data of heptane solvate D is shown in FIG. 22 of WO2013016491. Certain representative XRPD peaks of heptane solvate D are summarized in Table 23 below.

**Table 23: Certain representative XRPD of Heptane Solvate D**

| | Heptane Solvate D |
|---|---|
| **XRPD Peaks** | **Angle (2-Theta** ± **0.2)** |
| 1 | 10.3 |
| 2 | 13.7 |
| 3 | 16.9 |
| 4 | 18.9 |
| 5 | 19.3 |
| 6 | 20.4 |

5. Heptane Solvate E: 52.3 mg of Compound (1) was dispersed in 1 mL heptane. The suspension was stirred at room temperature for 5 days. The suspension was then filtered and analyzed by XRPD. Characteristics of heptane solvate E of Compound (1): XRPD data of heptane solvate E is shown in FIG. 23 of WO2013016491. Certain representative XRPD peaks of heptane solvate E are summarized in Table 24 below.

**Table 24: Certain representative XRPD of Heptane Solvate E**

| | Heptane Solvate D |
|---|---|
| **XRPD Peaks** | **Angle (2-Theta ± 0.2)** |
| 1 | 5.0 |
| 2 | 10.2 |
| 3 | 16.9 |
| 4 | 19.3 |
| 5 | 20.5 |
| 6 | 21.5 |

3L: Formation of MEK Solvates of Compound (1) (Compound (1) • MEK): MEK solvates of Compound (1) (Compound (1) • MEK can be prepared by following the steps described below: 400 mg of Compound (1) was added to 1 mL of MEK (methylethylketon (2-butanone)) in a vial. Thick slurry was obtained after vortexing the vial for 1 minute. The resulting mixture was then stirred for 4 hours. The solids from the wet slurry were analyzed by ¹³C SSNMR. Characteristics of MEK solvates of Compound (1): C SSNMR data of MEK solvates in FIG. 24 of WO2013016491. Certain representative peaks are summarized in Table 25 below.

**Table 25: Certain representative XRPD of Heptane Solvate A**

| ***¹³C SSNMR Shift*** | **ppm (± 0.2)** |
|---|---|
| 1 | 205,7 |
| 2 | 132.5 |
| 3 | 127.7 |
| 4 | 42.9 |
| 5 | 37.4 |
| 6 | 29.5 |
| 7 | 23.7 |
| 8 | 7.5 |

3M: Formation of Methylacetate Solvates of Compound (1) (Compound (1) • MeOAc): MeOAc solvates of Compound (1) (Compound (1) • MeOAc can be prepared by following the steps described below: 400 mg of Compound (1) was added to 1 mL of MeOAc in a vial. Thick slurry was obtained after vortexing the vial for 1 minute. The resulting mixture was then stirring for 4 hours. The solids from the wet slurry were analyzed by ¹³C SSNMR. Characteristics of MeOAc solvates of Compound (1): ¹³C SSNMR data of MeOAc solvates is shown in FIG. 25 of WO2013016491. Certain representative peaks are summarized in Table 26 below.

**Table 26: Certain representative XRPD of MeOAc Solvate**

| **¹³C SSNMR Shifts** | **ppm (± 0.2)** |
|---|---|
| 1 | 170.5 |
| 2 | 137.2 |
| 3 | 106.5 |
| 4 | 54.9 |
| 5 | 51.1 |
| 6 | 21.5 |
| 7 | 21.2 |

The invention relates to an administration unit comprising any of the solvates of Compound (1) according to the invention. The administration unit according to the invention is useful for treating various diseases and disorders which include but are not limited to inhibiting or reducing the activity of HCV polymerase in a biological in vitro sample or in a subject, treating a HCV infection in a subject.

### DEFINITION OF PREFERRED EMBODIMENTS OF ASPECTS (I) AND (II)

Preferably, the administration unit according to the invention comprises a therapeutically effective amount of Compound (1), wherein at least 10 % by weight of said therapeutically effective amount of Compound (1) are present as (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate. Preferably, at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of Compound (1) are present as (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate. Preferably, at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of Compound (1) are present as (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate. The total content of Compound (1) as well as the relative content of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate can be determined by standard analysis known to the skilled artisan. Suitable methods include but are not limited to thermal analysis, HPLC and XRPD.

In a preferred embodiment of the administration unit according to the invention, not more than 90 % by weight of said therapeutically effective amount of Compound (1) are amorphous. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of Compound (1) are amorphous. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of Compound (1) are amorphous.

The total content of Compound (1) as well as the relative content of amorphous (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate can be determined by standard analysis known to the skilled artisan. Suitable methods include but are not limited to thermal analysis, HPLC and XRPD (see e.g. K.D. Harris, Powder diffraction crystallography of molecular solids, Top Curr Chem., 2012, 315:133-77).

In a preferred embodiment of the administration unit according to the invention, not more than 90 % by weight of said therapeutically effective amount of Compound (1) are polymorphs of Compound (1) other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of Compound (1) are polymorphs of Compound (1) other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of Compound (1) are polymorphs of Compound (1) other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.

The total content of Compound (1) as well as the relative content of polymorphs other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate can be determined by standard analysis known to the skilled artisan. Suitable methods include but are not limited to thermal analysis, HPLC and XRPD (see e.g. N. Chieng, T. Rades, J.Aaltonen, An overview of recent studies on the analysis of pharmaceutical polymorphs, J Pharm Biomed Anal. 2011, 25:55(4):618-44; R. Hilfiker, Polymorphism, Wiley-VCH, 1st ed. 2006; H.G. Brittain, Polymorphism in Pharmaceutical Solids (Drugs and the Pharmaceutical Sciences), Informa Healthcare, 2nd ed. 2009).

In a preferred embodiment of the administration unit according to the invention, the therapeutically effective amount of Compound (1) is 0.1 µg to 2 g of Compound (1). Preferably, the therapeutically effective amount of Compound (1) is 1 µg to 2.5 µg of Compound (1), or 2.5 µg to 5 µg of Compound (1), or 5 µg to 10 µg of Compound (1), or 10 µg to 25 µg of Compound (1), or 25 µg to 50 µg of Compound (1), or 50 µg to 100 µg of Compound (1), or 100 µg to 250 µg of Compound (1), or 250 µg to 500 µg of Compound (1), or 500 µg to 1 mg of Compound (1), or 1 mg to 2.5 mg of Compound (1), or 2.5 mg to 5 mg of Compound (1), or 5 mg to 10 mg of Compound (1), or 10 mg to 25 mg of Compound (1), or 25 mg to 50 mg of Compound (1), or 50 mg to 100 mg of Compound (1), or 100 mg to 250 mg of Compound (1), or 250 mg to 500 mg of Compound (1), or 500 mg to 1 g of Compound (1), or 1 g to 1.25 g of Compound (1), or 1.25 g to 1.5 g of Compound (1), or 1.5 g to 1.75 g of Compound (1), or 1.75 g to 2 g of Compound (1).

Preferably, the administration unit according to the invention comprises 0.1 µg to 2 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate. Preferably, it comprises 1 µg to 2.5 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 2.5 µg to 5 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 5 µg to 10 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H20, Compound (1) • hydrate A, Compound (1) • hydrate B, Compound (1) • methanol, Compound (1) • thanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 10 µg to 25 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • thanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 25 µg to 50 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • thanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 50 µg to 100 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 100 µg to 250 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 250 µg to 500 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 500 µg to 1 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1 • mg to 2.5 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • thanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 2.5 mg to 5 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 5 mg to 10 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H20, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 10 mg to 25 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 25 mg to 50 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 50 mg to 100 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • thanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 100 mg to 250 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 250 mg to 500 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 500 mg to 1 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1 g to 1.25 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1.25 g to 1.5 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1.5 g to 1.75 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1.75 g to 2 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.

Preferably, the administration unit according to the invention contains (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate as substantially the only form of Compound (1), i.e. preferably contains neither non-crystalline forms of Compound (1) nor crystalline forms other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.

The administration unit according to the invention and its constituents, i.e. Compound (1) and (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, respectively, as well as pharmaceutical excipients, can be characterized by analytic methods that are known to the skilled artisan and described in Ph. Eur. and USP (see e.g. H.G. Brittain, S.J. Bodanowich, D.E. Bugay, J. DeVoncentis, G. Lewen, A.W. Newman, Physical characterization of pharmaceutical solids, Pharm Res. 1991, 8(8):963-73; D.E. Bugay, Characterization of the solid-state: spoectroscopic techniques, Adv Drug Deliv Rev., 2001, 48(1):43-65; P.A. Tishmack, D.E. Bugay, S.R. Byrn, Solid-state nuclear magnetic resonance spectroscopy - pharmaceutical application, J Pharm Sci, 2003, 92(3):441-74; C.J. Lee, C.J. Strachan, P.J. Manson, T. Rades, Characterization of the bulk properties of pharmaceutical solids using nonlinear optics - a review, J Pharm Pharmacol., 2007, 59(2):241-50); R.A. Storey, Solid State Characterization of Pharmaceuticals, Wiley-Blackwell, 2011).

In a preferred embodiment, the administration unit according to the invention contains (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • thanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate in form of particles, wherein the particle size distribution of X90 is about 500 µm or less, i.e. a particle size distribution (preferably by volume) such that at least 90 % of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate has a particle size smaller than 500 µm. Preferably, for (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the administration unit according to the invention contains (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate in form of particles, wherein the particle size distribution of X50 is about 400 µm or less, i.e. a particle size distribution (preferably by volume) such that at least 50 % of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate has a particle size smaller than 400 µm. Preferably, for (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

According to USP, the following parameters may be defined based on the cumulative distribution. QR(X) = cumulative distribution of particles with a dimension less than or equal to X [µm] where the subscript R reflects the distribution type: 0 Number, 1 Length, 2 Area, 3 Volume. Therefore, by definition: QR(X) = 0.90 when X = X90; QR(X) = 0.50 when X = X50; and QR(X) = 0.10 when X = X10.

In preferred embodiments, the administration unit according to the invention contains (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate in form of particles having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50. In preferred embodiments, the administration unit according to the invention contains (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate in form of particles having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the administration unit according to the invention contains (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate in form of particles having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Preferably, the administration unit according to the invention is solid, semisolid or liquid.

Preferably, the administration unit according to the invention is for administration once daily, or twice daily, or thrice daily, or four times daily, or five times daily, or six times daily.

Preferably, the administration unit according to the invention is monolithic or multiparticulate.

In a preferred embodiment of the administration unit according to the invention, (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate is homogeneously distributed over the administration unit.

Excipient composition and homogeneity can be determined by standard analysis known to the skilled artisan. Suitable methods include but are not limited to near-infrared chemical imaging.

In another preferred embodiment of the administration unit according to the invention, (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate is not homogeneously distributed over the administration unit.

In a preferred embodiment, the administration unit according to the invention provides controlled release of Compound (1). Preferably, the administration unit according to the invention comprises a controlled release matrix or a controlled release coating.

In a preferred embodiment, under in vitro conditions (paddle apparatus, 75 rpm, 900 mL artificial gastric juice, pH 1.2) the administration unit according to the invention has released after 1 hour not more than 90 % of Compound (1). Preferably, the administration unit according to the invention has released after 1 hour not more than 80 % of Compound (1), or not more than 70 % of Compound (1), or not more than 60 % of Compound (1), or not more than 50 % of Compound (1), or not more than 40 % of Compound (1), or not more than 30 % of Compound (1), or not more than 20 % of Compound (1), or not more than 10 % of Compound (1).

In a preferred embodiment, under in vitro conditions (paddle apparatus, 75 rpm, 900 mL artificial gastric juice, pH 1.2) the administration unit according to the invention has released after 2 hours not more than 90 % of Compound (1). Preferably, the administration unit according to the invention has released after 2 hours not more than 80 % of Compound (1), or not more than 70 % of Compound (1), or not more than 60 % of Compound (1), or not more than 50 % of Compound (1), or not more than 40 % of Compound (1), or not more than 30 % of Compound (1), or not more than 20 % of Compound (1), or not more than 10 % of Compound (1).

In a preferred embodiment, under in vitro conditions (paddle apparatus, 75 rpm, 900 mL artificial gastric juice, pH 1.2) the administration unit according to the invention has released after 4 hours not more than 90 % of Compound (1). Preferably, the administration unit according to the invention has released after 4 hours not more than 80 % of Compound (1), or not more than 70 % of Compound (1), or not more than 60 % of Compound (1), or not more than 50 % of Compound (1), or not more than 40 % of Compound (1), or not more than 30 % of Compound (1), or not more than 20 % of Compound (1), or not more than 10 % of Compound (1).

In a preferred embodiment, under in vitro conditions (paddle apparatus, 75 rpm, 900 mL artificial gastric juice, pH 1.2) the administration unit according to the invention has released after 8 hours not more than 90 % of Compound (1). Preferably, the administration unit according to the invention has released after 8 hours not more than 80 % of Compound (1), or not more than 70 % of Compound (1), or not more than 60 % of Compound (1), or not more than 50 % of Compound (1), or not more than 40 % of Compound (1), or not more than 30 % of Compound (1), or not more than 20 % of Compound (1), or not more than 10 % of Compound (1).

In another preferred embodiment, the administration unit according to the invention provides immediate release of Compound (1).

In a preferred embodiment, under in vitro conditions (paddle apparatus, 75 rpm, 900 mL artificial gastric juice, pH 1.2) the administration unit according to the invention has released after 0.5 hours at least 10 % of Compound (1). Preferably, the administration unit according to the invention has released after 0.5 hours at least 20 % of Compound (1), or at least 30 % of Compound (1), or at least 40 % of Compound (1), or at least 50 % of Compound (1), or at least 60 % of Compound (1), or at least 70 % of Compound (1), or at least 80 % of Compound (1), or at least 90 % of Compound (1).

Preferably, the administration unit according to the invention has a total weight of 10 mg to 3 g. Preferably, the administration unit according to the invention has a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

In a preferred embodiment, the administration unit according to the invention is for systemic or topical administration.

In another preferred embodiment, the administration unit according to the invention is for parenteral administration. Preferably, the administration unit according to the invention is foral, buccal, opthalmic, nasal, rectal, transmucosal, or intestinal administration. Preferably, it is foral administration.

Preferably, the administration unit according to the invention is selected from the group consisting of tablets, capsules, effervescent tablets, orodispersible tablets, dragees, sachets, drops, suspensions, and powders. Preferably, it is a tablet. Preferably, the administration unit according to the invention is round or oblong; and/or is planar or biconvex.

In a preferred embodiment, the administration unit according to the invention has a round cross-section with a diameter of 1 mm to 20 mm. Preferably, the diameter of the round cross-section is 1 mm to 3 mm, or 3 mm to 5 mm, or 5 mm to 8 mm, or 8 mm to 10 mm, or 10 mm to 13 mm, or 13 mm to 15 mm, or 15 mm to 18 mm, or 18 mm to 20 mm.

In a preferred embodiment, the administration unit according to the invention is oblong and has an aspect ratio of at least 1.1:1. Preferably, the aspect ratio is at least 4:3, or at least 21/2:1, or at least 3:2, or at least 16:10, or at least □:1, or at least 5:3, or at least 16:9.

In a preferred embodiment, the administration unit according to the invention has been manufactured by direct compression. In another preferred embodiment, the administration unit according to the invention has been manufactured by granulation and subsequent compression of granules. Preferably, granulation is wet granulation or dry granulation.

Preferably, the administration unit according to the invention has been compacted at a pressure of 10 MPa to 10,000 MPa. Preferably, the administration unit according to the invention has been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

In a preferred embodiment, the administration unit according to the invention comprises a film coating. Preferably, the film coating is enteric. Suitable enteric coating materials are known to the skilled artisan and include but are not limited to methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In a preferred embodiment of the administration unit according to the invention, the film coating comprises a cellulose ether (e.g. hydroxypropylmethyl cellulose) or a synthetic polymer (e.g. polyvinyl alcohol).

In a preferred embodiment of the administration unit according to the invention, the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

In a preferred embodiment, the administration unit according to the invention has a tablet porosity of not more than 90 %. Preferably, it has a tablet porosity of not more than 80 %, or not more than 70 %, or not more than 60 %, or not more than 50 %, or not more than 40 %, or not more than 30 %, or not more than 20 %, or not more than 10 %. Preferably, it has a tablet porosity of not more than 5 %, or not more than 4 %, or not more than 3 %, or not more than 2 %, or not more than 1 %, or not more than 0.5 %, or not more than 0.2 %, or not more than 0.1 %.

The most common strength test in pharmaceutical applications is the diametral compression test, which is used to calculate the radial tensile strength of a tablet (Fell, J.T., Newton, J.M., 1970. Determination of tablet strength by diametral compression test. J. Pharm. Sci. 59, 688-691). In order to calculate the radial tensile strength, the stress conditions have to be such that the tablet fails in tension. During radial tensile strength measurements, the fracture occurs through a predetermined diametral cross section of the tablet. Therefore, the radial tensile strength is likely to reflect the average strength of tablet rather than the strength of the weakest plane in the tablet.

In a preferred embodiment, the administration unit according to the invention has a radial tensile strength of 0.1 to 100 MPa. Preferably, it has a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

Another method for determining mechanical strength is to measure the axial tensile strength. The force necessary to break the tablet is obtained by pulling the tablet parallel to the applied force during the formation of the tablet and this force is then used to calculate the axial tensile strength (Nyström, C., Malmqvist, K., Mazur, J., Alex, W., Hölzer, A.W., 1978. Measurement of axial and radial tensile strength of tablets and their relation to capping. Acta Pharm. Suec. 15, 226-232). During axial tensile strength measurements, the fracture will occur through the weakest plane in the tablet. Consequently, this method allows detection of capping tendencies in a tablet.

In a preferred embodiment, the administration unit according to the invention has an axial tensile strength of 0.1 to 100 MPa. Preferably, it has an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

In a preferred embodiment, the administration unit according to the invention has an average pore size of 0.001 µm to 1000 µm. Preferably, it has an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to 1 µm, or 1 µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

The pore size distribution of a tablet may be assessed by methods that are known to the skilled artisan and include but are not limited to gas adsorption (e.g. Stanley-Wood, N.G., Johansson, M.E., 1980. Variation of intra- and inter-particle porosity with degree of compaction. Analyst 105, 1104-1112; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86) or mercury porosimetry (e.g. Stanley-Wood, N.G., Johansson, M.E., 1980. Variation of intra- and inter-particle porosity with degree of compaction. Analyst 105, 1104-1112; Juppo, A.M., 1996. Relationship between breaking force and pore structure of lactose, glucose and mannitol tablets. Int. J. Pharm.127, 95-102; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86). These techniques are complementary, in that mercury porosimetry can be used to measure larger pores (the lower size limit is about 0.003 µm in diameter) while gas adsorption allows measurement of smaller pores. For further details it is also referred to S. Lowell et al., Characterization of Porous Solids and Powders: Surface Area, Pore Size and Density (Particle Technology Series), Springer, 2010.

In a preferred embodiment, the administration unit according to the invention has a pore size distribution such that at least 10 % of the pores have a pore size of not more than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 0.1 µm.

In another preferred embodiment, the administration unit according to the invention has a pore size distribution such that at least 10 % of the pores have a pore size of not less than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 0.1 µm.

In preferred embodiment, the administration unit according to the invention has a pore size distribution such that at least 10 % of the pores have a pore size of not more than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 1 µm.

In another preferred embodiment, the administration unit according to the invention has a pore size distribution such that at least 10 % of the pores have a pore size of not less than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 1 µm.

In a preferred embodiment, the administration unit according to the invention has a pore size distribution such that at least 10 % of the pores have a pore size of not more than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 10 µm.

In another preferred embodiment, the administration unit according to the invention has a pore size distribution such that at least 10 % of the pores have a pore size of not less than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 10 µm.

In a preferred embodiment, the administration unit according to the invention has a water content of not more than 5 % by weight, relative to the total weight of the tablet. Preferably, it has a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

In a preferred embodiment, the administration unit according to the invention has a true density of 0.60 to 1.40 g cm-3. Preferably, it has a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention has an apparent density of 0.60 to 1.40 g cm-3. Preferably, it has an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention disintegrates within 6000 seconds. Preferably, it disintegrates within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

In a preferred embodiment, the administration unit according to the invention is a capsule. Preferably, it comprises a multitude of particulates comprising (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate. Preferably, it comprises at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

In a preferred embodiment of the administration unit according to the invention, the capsule material comprises hard gelatine. In a preferred embodiment, the administration unit according to the invention comprises at least 2 different types of particulates that differ from each other in at least one property selected from the group consisting of nature of excipients, content of excipients, content of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, content of Compound (1), water content, total weight, size, and shape.

In a preferred embodiment, the administration unit according to the invention passes the storage stability test according to Ph. Eur. under accelerated storage conditions at 40°C and 75 % rel. humidity. Preferably, it has a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

In a preferred embodiment, the administration unit according to the invention provides the peak plasma level within 0.1 hour to 36 hours after administration. Preferably, it provides the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

Preferably, the administration unit according to the invention comprises one or more excipients independently selected from the group consisting of antiadherents, binders, disintegrants, fillers, diluents, flavors, colors, lubricants, glidants, sorbents, surfactants, preservatives and sweeteners.

In a preferred embodiment of the administration unit according to the invention, the antiadherent is selected from the group consisting of silicon dioxide, talc, talc waxes, stearates (e.g. magnesium, calcium and sodium), stearic acid, stearowet, boric acid, sodium chloride, DL-leucine, sodium oleate, sodium benzoate, sodium acetate, sodium lauryl sulfate, and magnesium lauryl sulfate. Magnesium stearate is particularly preferred.

In a preferred embodiment of the administration unit according to the invention, the binder is selected from the group consisting of saccharides and their derivatives; proteins; gums; silicates; and synthetic polymers. Preferably, the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

In a preferred embodiment of the administration unit according to the invention, the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

In a preferred embodiment of the administration unit according to the invention, the filler (or diluent) is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

In a preferred embodiment of the administration unit according to the invention, the lubricant is hydrophilic or hydrophobic. Preferably, the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

In a preferred embodiment of the administration unit according to the invention, the glidant is selected from the group consisting of fumed silica, cornstarch, talc, and magnesium carbonate.

In a preferred embodiment of the administration unit according to the invention, the surfactant is selected from the group consisting of sodium lauryl sulfate, polyethylene-polypropylene glycol co-polymers, poly(oxyethylene)-poly(oxypropylene)-block-copolymers (e.g. poloxamers).

In a preferred embodiment of the administration unit according to the invention, the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

In a preferred embodiment, the administration unit according to the invention passes the friability test according to Ph. Eur. Preferably, the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

In a preferred embodiment, the administration unit according to the invention passes the test uniformity of content of single-dose preparations according to Ph. Eur.

In a preferred embodiment, the administration unit according to the invention does not comprise any active pharmaceutical ingredient other than Compound (1).

In a preferred embodiment, the administration unit according to the invention is for storage not above 50 °C. Preferably, it is for storage not above 25 °C.

In a preferred embodiment, the administration unit according to the invention is not identical with any of the administration units that are individualized in the experimental section of WO 2013/016490, WO 2013/016491, WO 2013/016492, WO 2013/016499, and WO 2013/016501, which is incorporated by reference.

The invention also relates to the administration unit according to the invention for use in the treatment of a disorder or a disease in a mammal. Preferably, the mammal is a human. Preferably, the mammal is an adult.

Preferably, the mammal does not suffer from liver damage and/or kidney damage.

Preferably, the mammal is not pregnant.

Preferably, the mammal is not allergic against Compound (1).

In a preferred embodiment, the administration unit according to the invention is administered orally with a liquid. Preferably, the liquid is water, milk, juice or lemonade.

In a preferred embodiment of the administration unit according to the invention, the administered daily dose of Compound (1) is 0.0001 mg/kg body weight to 100 mg/kg body weight. Preferably, the administered daily dose of Compound (1) is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the administration unit according to the invention, the administered daily dose of Compound (1) is 0.001 mg to 5000 mg. Preferably, the administered daily dose of Compound (1) is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

The invention also relates to a packaging comprising one or more administration units according to the invention.

In a preferred embodiment, the packaging according to the invention comprises a material selected from the group consisting of paper, cardboard, paperboard, metal foil and plastic foil.

In a preferred embodiment, the packaging according to the invention is a blister packaging.

A preferred blister packaging includes but is not limited to PVC-blister, PVDC-blister, PVC/PVDC-blister, moisture-proof packaging material such as aluminium foil blister pack, alu/alu blister, transparent or opaque polymer blister with pouch.

An alternative packaging according to the invention is a polypropylene tube, glass bottle and HDPE bottle optionally containing a child-resistant feature. The primary packaging material may comprise a desiccant such as molecular sieve or silica gel to improve chemical stability of Compound (1). Opaque packaging such as colored blister materials, tubes, brown glass bottles or the like can be used to prolong shelflife of Compound (1) by reduction of photodegradation.

In a preferred embodiment, the packaging according to the invention comprises a multitude of at least 2, or at least 3, or at least 4, or at least 5, or at least 6, or at least 7, or at least 8, or at least 9, or at least 10 administration units according to the invention.

In a preferred embodiment of the packaging according to the invention, the administration units do not substantially differ in at least one property selected from the group consisting of nature of excipients, content of excipients, content of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, content of Compound (1), water content, total weight, release profile, disintegration time, size, shape, porosity, apparent density, true density, average pore size, pore size distribution, axial tensile strength, radial tensile strength, friability, and storage stability. Preferably, the administration units differ in said at least one property by not more than 5 %, or not more than 4 %, or not more than 3 %, or not more than 2 %, or not more than 1 %, or not more than 0.5 %, or not more than 0.1 %. Preferably, all administration units are substantially identical.

The invention also relates to the packaging according to the invention for use in the treatment of a disorder or a disease, wherein one or more administration units of said multitude are administered on a first day, and one or more administration units of said multitude are administered on a second day following said first day.

In a preferred embodiment of the packaging according to the invention, one or more administration units of said multitude are administered on one or more subsequent days following said second day. Preferably, the time interval between the administration of an administration unit and the administration of a subsequent administration unit is not more than 48 hours. Preferably, the time interval is not more than 36 hours, or not more than 24 hours, or not more than 12 hours, or not more than 8 hours, or not more than 6 hours, or not more than 4 hours.

In a preferred embodiment of the packaging according to the invention, the administration units are administered on not more than 30 subsequent days. Preferably, the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.

In a preferred embodiment of the packaging according to the invention, the administered daily dose of Compound (1) is 0.0001 mg/kg body weight to 100 mg/kg body weight. Preferably, the administered daily dose of Compound (1) is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight. In a preferred embodiment of the packaging according to the invention, the administered daily dose of Compound (1) is 0.001 mg to 5000 mg. Preferably, the administered daily dose of Compound (1) • is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

In a preferred embodiment, the packaging according to the invention does not comprise any administration unit comprising an active pharmaceutical ingredient other than Compound (1).

The invention also relates to a method for manufacturing an administration unit according to the invention or a packaging according to the invention, comprising the steps:
(a) providing a quantity of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate that exceeds the dose of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate to be contained in a single administration unit by a factor of at least 10;
(b) mixing the quantity of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate with one or more pharmaceutical excipients;
(c) dividing the mixture obtained in step (b) in fractions each containing the dose of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate to be contained in a single administration unit;
(d) optionally, forming the fractions obtained in step (c) to administration units; and
(e) optionally, packing the fractions obtained in step (c) and the administration units obtained in step (d), respectively. In a preferred embodiment of the method according to the invention, in step (a) the quantity of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate exceeds the dose of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate to be contained in a single administration unit by a factor of at least 25, or at least 50, or at least 100, or at least 250, or at least 500, or at least 1000, or at least 2500, or at least 5000, or at least 10,000.

The invention also relates to particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate having a particle size distribution X90 of about 500 µm or less, i.e. a particle size distribution (preferably by volume) such that at least 90 % of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate has a particle size smaller than 500 µm. Preferably, for (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to the invention have a particle size distribution X50 of about 400 µm or less, i.e. a particle size distribution (preferably by volume) such that at least 50 % of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate has a particle size smaller than 400 µm. Preferably, for (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

In preferred embodiments, the particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate have a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate have a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate have a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Suitable milling and grinding techniques that are suitable for obtaining a specific particle size distribution are known to the skilled artisan (see e.g. N. Rasenack, B.W. Müller, Micron-size drug particles: common and novel micronization techniques, Pharm Dev Technol., 2004, 9(1):1-13; A. Martin, M.J. Cocero, Micronization processes with supercritical fluids: fundamentals and mechanisms, Adv Drug Deliv Rev. 2008, 60(3):339-50; S.C. Gad, Pharmaceutical Manufacturing Handbook: Production and Processes (Pharmaceutical Development Series), Wiley Interscience, 1st ed. 2008; A.J. Hickey, Pharmaceutical Process Engineering (Drugs and the Pharmaceutical Sciences), Informa Healthcare, 2nd ed. 2009; B. Nickerson, Sample Preparation of Pharmaceutical Dosage Forms: Challenges and Strategies for Sample Preparation and Extraction, Springer, 1st ed. 2011; D. Dragoman, Optical Characteriaztion of Solids, Springer, 1st ed. 2010; and D. Schulze; Powders and Bulk Solids: Behavior, Characterization, Storage and Flow, Springer, 2008).

### PREFERRED EMBODIMENTS OF ASPECTS (I) AND (II) DEFINED BY ITEMS 1 TO 137

Preferred embodiments of the invention are items 1 to items 137:
1. An administration unit comprising (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
2. The administration unit according to item 1, comprising a therapeutically effective amount of Compound (1), wherein at least 10 % by weight of said therapeutically effective amount of Compound (1) are present as (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
3. The administration unit according to item 2, wherein at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of Compound (1) • are present as (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
4. The administration unit according to any of items 2 to 3, wherein at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of Compound (1) are present as (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
5. The administration unit according to any of items 2 to 4, wherein not more than 90 % by weight of said therapeutically effective amount of Compound (1) are amorphous.
6. The administration unit according to item 5, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of Compound (1) are amorphous.
7. The administration unit according to item 5 or 6, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of Compound (1) are amorphous.
8. The administration unit according to any of items 2 to 7, wherein not more than 90 % by weight of said therapeutically effective amount of Compound (1) are polymorphs of Compound (1) other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
9. The administration unit according to item 8, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of Compound (1) • are polymorphs of Compound (1) • other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
10. The administration unit according to item 8 or 9, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of Compound (1) are polymorphs of Compound (1) other than (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
11. The administration unit according to any of items 2 to 10, wherein the therapeutically effective amount of Compound (1) is 0.1 µg to 2 g of Compound (1).
12. The administration unit according to item 11, wherein the therapeutically effective amount of Compound (1) is 1 µg to 2.5 µg of Compound (1), or 2.5 µg to 5 µg of Compound (1), or 5 µg to 10 µg of Compound (1), or 10 µg to 25 µg of Compound (1), or 25 µg to 50 µg of Compound (1), or 50 µg to 100 µg of Compound (1), or 100 µg to 250 µg of Compound (1), or 250 µg to 500 µg of Compound (1), or 500 µg to 1 mg of Compound (1), or 1 mg to 2.5 mg of Compound (1), or 2.5 mg to 5 mg of Compound (1), or 5 mg to 10 mg of Compound (1), or 10 mg to 25 mg of Compound (1), or 25 mg to 50 mg of Compound (1), or 50 mg to 100 mg of Compound (1), or 100 mg to 250 mg of Compound (1), or 250 mg to 500 mg of Compound (1), or 500 mg to 1 g of Compound (1), or 1 g to 1.25 g of Compound (1), or 1.25 g to 1.5 g of Compound (1), or 1.5 g to 1.75 g of Compound (1), or 1.75 g to 2 g of Compound (1).
13. The administration unit according to any of items 1 to 12, comprising 0.1 µg to 2 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
14. The administration unit according to item 13, comprising 1 µg to 2.5 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 2.5 µg to 5 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 5 µg to 10 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 10 µg to 25 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 25 µg to 50 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 50 µg to 100 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 100 µg to 250 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 250 µg to 500 µg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 500 µg to 1 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1 mg to 2.5 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 2.5 mg to 5 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 5 mg to 10 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 10 mg to 25 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 25 mg to 50 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 50 mg to 100 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 100 mg to 250 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 250 mg to 500 mg of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 500 mg to 1 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1 g to 1.25 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1.25 g to 1.5 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1.5 g to 1.75 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, or 1.75 g to 2 g of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
15. The administration unit according to any of items 1 to 14, which is solid, semisolid or liquid.
16. The administration unit according to any of items 1 to 15, which is for administration once daily, or twice daily, or thrice daily, or four times daily, or five times daily, or six times daily.
17. The administration unit according to any of items 1 to 16, which is monolithic or multiparticulate.
18. The administration unit according to any of items 1 to 17, wherein (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate is homogeneously distributed over the administration unit.
19. The administration unit according to any of items 1 to 17, wherein (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate is not homogeneously distributed over the administration unit.
20. The administration unit according to any of items 1 to 19, providing controlled release of Compound (1).
21. The administration unit according to item 20, comprising a controlled release matrix or a controlled release coating.
22. The administration unit according to any of items 1 to 19, providing immediate release of Compound (1).
23. The administration unit according to any of items 1 to 22, having a total weight of 10 mg to 3 g.
24. The administration unit according to item 23, having a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.
25. The administration unit according to any of items 1 to 24, which is for systemic or topical administration.
26. The administration unit according to any of items 1 to 25, which is for parenteral administration.
27. The administration unit according to any of items 1 to 26, which is foral, buccal, opthalmic, nasal, rectal, transmucosal, or intestinal administration.
28. The administration unit according to any of items 1 to 27, which is foral administration.
29. The administration unit according to item 28, which is selected from the group consisting of tablets, capsules, effervescent tablets, orodispersible tablets, dragees, sachets, drops, suspensions, and powders.
30. The administration unit according to any of items 1 to 29, which is a tablet.
31. The administration unit according to item 30, which is round or oblong; and/or which is planar or biconvex.
32. The administration unit according to any of items 30 to 31, having been manufactured by direct compression.
33. The administration unit according to any of items 30 to 31, having been manufactured by granulation and subsequent compression of granules.
34. The administration unit according to item 33, wherein granulation is wet granulation or dry granulation.
35. The administration unit according to any of items 30 to 34, having been compacted at a pressure of 10 MPa to 10,000 MPa.
36. The administration unit according to item 35, having been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.
37. The administration unit according to any of items 30 to 36, comprising a film coating.
38. The administration unit according to item 37, wherein the film coating is enteric.
39. The administration unit according to any of items 37 to 38, wherein the film coating comprises a cellulose ether (e.g. hydroxypropylmethyl cellulose) or a synthetic polymer (e.g. polyvinyl alcohol).
40. The administration unit according to any of items 37 to 39, wherein the film coating has an average thickness of 0.01 to 1000 µm.
41. The administration unit according to item 40, wherein the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 µm, or 500 µm to 1000 µm.
42. The administration unit according to any of items 30 to 41, having a tablet porosity of not more than 90 %.
43. The administration unit according to item 42, having a tablet porosity of not more than 80 %, or not more than 70 %, or not more than 60 %, or not more than 50 %, or not more than 40 %, or not more than 30 %, or not more than 20 %, or not more than 10 %.
44. The administration unit according to item 42 to 43, having a tablet porosity of not more than 5 %, or not more than 4 %, or not more than 3 %, or not more than 2 %, or not more than 1 %, or not more than 0.5 %, or not more than 0.2 %, or not more than 0.1 %.
45. The administration unit according to any of items 30 to 44, having a radial tensile strength of 0.1 to 100 MPa.
46. The administration unit according to item 45, having a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
47. The administration unit according to any of items 30 to 46, having an axial tensile strength of 0.1 to 100 MPa.
48. The administration unit according to item 47, having an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
49. The administration unit according to any of items 30 to 48, having an average pore size of 0.001 µm to 1000 µm.
50. The administration unit according to item 49, having an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to 1 µm, or 1 µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.
51. The administration unit according to any of items 30 to 50, having a pore size distribution such that at least 10 % of the pores have a pore size of not more than 0.1 µm.
52. The administration unit according to item 51, having a pore size distribution such that at least 20 %, or at least 30 %, or at least 40 %, or at least 50 %, or at least 60 %, or at least 70 %, or at least 80 %, or at least 90 % of the pores have a pore size of not more than 0.1 µm.
53. The administration unit according to any of items 30 to 52, having a pore size distribution such that at least 10 % of the pores have a pore size of not less than 0.1 µm.
54. The administration unit according to item 53, having a pore size distribution such that at least 20 %, or at least 30 %, or at least 40 %, or at least 50 %, or at least 60 %, or at least 70 %, or at least 80 %, or at least 90 % of the pores have a pore size of not less than 0.1 µm.
55. The administration unit according to any of items 30 to 54, having a pore size distribution such that at least 10 % of the pores have a pore size of not more than 1 µm.
56. The administration unit according to item 55, having a pore size distribution such that at least 20 %, or at least 30 %, or at least 40 %, or at least 50 %, or at least 60 %, or at least 70 %, or at least 80 %, or at least 90 % of the pores have a pore size of not more than 1 µm.
57. The administration unit according to any of items 30 to 56, having a pore size distribution such that at least 10 % of the pores have a pore size of not less than 1 µm.
58. The administration unit according to item 57, having a pore size distribution such that at least 20 %, or at least 30 %, or at least 40 %, or at least 50 %, or at least 60 %, or at least 70 %, or at least 80 %, or at least 90 % of the pores have a pore size of not less than 1 µm.
59. The administration unit according to any of items 30 to 58, having a pore size distribution such that at least 10 % of the pores have a pore size of not more than 10 µm.
60. The administration unit according to item 59, having a pore size distribution such that at least 20 %, or at least 30 %, or at least 40 %, or at least 50 %, or at least 60 %, or at least 70 %, or at least 80 %, or at least 90 % of the pores have a pore size of not more than 10 µm.
61. The administration unit according to any of items 30 to 60, having a pore size distribution such that at least 10 % of the pores have a pore size of not less than 10 µm.
62. The administration unit according to item 61, having a pore size distribution such that at least 20 %, or at least 30 %, or at least 40 %, or at least 50 %, or at least 60 %, or at least 70 %, or at least 80 %, or at least 90 % of the pores have a pore size of not less than 10 µm.
63. The administration unit according to any of items 30 to 62, having a water content of not more than 5 % by weight, relative to the total weight of the tablet.
64. The administration unit according to item 63, having a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.
65. The administration unit according to any of items 30 to 64, having a true density of 0.60 to 1.40 g cm-3.
66. The administration unit according to item 65, having a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
67. The administration unit according to any of items 30 to 66, having an apparent density of 0.60 to 1.40 g cm-3.
68. The administration unit according to item 67, having an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
69. The administration unit according to any of items 30 to 68, disintegrating within 6000 seconds.
70. The administration unit according to item 69, disintegrating within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.
71. The administration unit according to any of items 1 to 29, which is a capsule.
72. The administration unit according to item 71, comprising a multitude of particulates comprising (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate.
73. The administration unit according to any of items 71 to 72, comprising at least 2 particulates, or at least 3 particulates, or at least 4 particulates, or at least 5 particulates, or at least 6 particulates, or at least 7 particulates, or at least 8 particulates, or at least 9 particulates, or at least 10 particulates.
74. The administration unit according to any of items 71 to 73, wherein the capsule material comprises hard gelatine.
75. The administration unit according to any of items 71 to 74, comprising at least 2 different types of particulates that differ from each other in at least one property selected from the group consisting of nature of excipients, content of excipients, content of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, content of Compound (1), water content, total weight, size, and shape.
76. The administration unit according to any of items 1 to 75, passing the storage stability test according to Ph. Eur. under accelerated storage conditions at 40°C and 75 % rel. humidity.
77. The administration unit according to item 76, having a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.
78. The administration unit according to any of items 1 to 77, providing the peak plasma level within 0.1 hour to 36 hours after administration.
79. The administration unit according to item 78, providing the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.
80. The administration unit according to any of items 1 to 79, comprising one or more excipients independently selected from the group consisting of antiadherents, binders, disintegrants, fillers, flavors, colors, lubricants, glidants, sorbents, surfactants, preservatives and sweeteners.
81. The administration unit according to item 80, wherein the antiadherent is selected from the group consisting of silicon dioxide, talc, talc waxes, stearates (e.g. magnesium, calcium or sodium), stearic acid, stearowet, boric acid, sodium chloride, DL-leucine, sodium oleate, sodium benzoate, sodium acetate, sodium lauryl sulfate, and magnesium lauryl sulfate.
82. The administration unit according to any of items 80 to 81, wherein the binder is selected from the group consisting of saccharides and their derivatives; proteins; gums; silicates; and synthetic polymers.
83. The administration unit according to item 82, wherein the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.
84. The administration unit according to any of items 80 to 83, wherein the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).
85. The administration unit according to any of items 80 to 84, wherein the filler is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).
86. The administration unit according to any of items 80 to 85, wherein the lubricant is hydrophilic or hydrophobic.
87. The administration unit according to item 86, wherein the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.
88. The administration unit according to any of items 80 to 87, wherein the glidant is selected from the group consisting of fumed silica, cornstarch, talc, and magnesium carbonate.
89. The administration unit according to any of items 80 to 88, wherein the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.
90. The administration unit according to any of items 1 to 89, passing the friability test according to Ph. Eur.
91. The administration unit according to item 90, wherein the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.
92. The administration unit according to any of items 1 to 91, passing the test uniformity of content of single-dose preparations according to Ph. Eur.
93. The administration unit according to any of items 1 to 92, not comprising any active pharmaceutical ingredient other than Compound (1).
94. The administration unit according to any of items 1 to 93, which is for storage not above 50 °C.
95. The administration unit according to item 94, which is for storage not above 25 °C.
96. The administration unit according to any of items 1 to 95, with the proviso that the administration unit is not identical with any of the administration units that are individualized in the experimental section of WO 2013/016490, WO 2013/016491, WO 2013/016492, WO 2013/016499, and WO 2013/016501.
97. The administration unit according to any of items 1 to 96 for use in the treatment of a disorder or a disease in a mammal.
98. The administration unit according to item 97, wherein the mammal is a human.
99. The administration unit according to any of items 97 to 98, wherein the mammal is an adult.
100. The administration unit according to any of items 97 to 99 wherein the mammal does not suffer from liver damage.
101. The administration unit according to any of items 97 to 100, wherein the mammal does not suffer from kidney damage.
102. The administration unit according to any of items 97 to 101, wherein the mammal is not pregnant.
103. The administration unit according to any of items 97 to 102, wherein the mammal is not allergic against Compound (1).
104. The administration unit according to any of items 97 to 103, which is administered orally with a liquid.
105. The administration unit according to item 104, wherein the liquid is water, milk, juice or lemonade.
106. The administration unit according to any of items 97 to 105, wherein the administered daily dose of Compound (1) is 0.0001 mg/kg body weight to 100 mg/kg body weight.
107. The administration unit according to item 106, wherein the administered daily dose of Compound (1) is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
108. The administration unit according to any of items 97 to 107, wherein the administered daily dose of Compound (1) is 0.001 mg to 5000 mg.
109. The administration unit according to item 108, wherein the administered daily dose of Compound (1) is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 1999 mg.
110. A packaging comprising one or more administration units according to any of items 1 to 109.
111. The packaging according to item 110, comprising a material selected from the group consisting of paper, cardboard, paperboard, metal foil and plastic foil.
112. The packaging according to any of items 110 to 111, which is a blister packaging.
113. The packaging according to any of items 110 to 112, comprising a multitude of at least 2, or at least 3, or at least 4, or at least 5, or at least 6, or at least 7, or at least 8, or at least 9, or at least 10 administration units according to any of items 1 to 109.
114. The packaging according to item 113, wherein the administration units do not substantially differ in at least one property selected from the group consisting of nature of excipients, content of excipients, content of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate, content of Compound (1), water content, total weight, release profile, disintegration time, size, shape, porosity, apparent density, true density, average pore size, pore size distribution, axial tensile strength, radial tensile strength, friability, and storage stability.
115. The packaging according to item 114, wherein the administration units differ in said at least one property by not more than 5 %, or not more than 4 %, or not more than 3 %, or not more than 2 %, or not more than 1 %, or not more than 0.5 %, or not more than 0.1 %.
116. The packaging according to any of items 113 to 115, wherein all administration units are substantially identical.
117. The packaging according to any of items 113 to 116 for use in the treatment of a disorder or a disease, wherein one or more administration units of said multitude are administered on a first day, and one or more administration units of said multitude are administered on a second day following said first day.
118. The packaging according to item 117, wherein one or more administration units of said multitude are administered on one or more subsequent days following said second day.
119. The packaging according to any of items 117 to 118, wherein the time interval between the administration of an administration unit and the administration of a subsequent administration unit is not more than 48 hours.
120. The packaging according to item 119, wherein the time interval is not more than 36 hours, or not more than 24 hours, or not more than 12 hours, or not more than 8 hours, or not more than 6 hours, or not more than 4 hours.
121. The packaging according to any of items 117 to 120, wherein the administration units are administered on not more than 30 subsequent days.
122. The packaging according to item 121, wherein the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.
123. The packaging according to any of items 117 to 122, wherein the administered daily dose of Compound (1) is 0.0001 mg/kg body weight to 100 mg/kg body weight.
124. The packaging according to item 123, wherein the administered daily dose of Compound (1) is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
125. The packaging according to any of items 117 to 124, wherein the administered daily dose of Compound (1) is 0.001 mg to 5000 mg.
126. The packaging according to item 125, wherein the administered daily dose of Compound (1) is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.
127. The packaging according to any of items 117 to 126, not comprising any administration unit comprising an active pharmaceutical ingredient other than Compound (1).
128. A method for manufacturing an administration unit according to any of items 1 to 109 or a packaging according to any of items 110 to 127, comprising the steps: (a) providing a quantity of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate that exceeds the dose of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate to be contained in a single administration unit by a factor of at least 10; (b) mixing the quantity of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate with one or more pharmaceutical excipients; (c) dividing the mixture obtained in step (b) in fractions each containing the dose of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate to be contained in a single administration unit; (d) optionally, forming the fractions obtained in step (c) to administration units; and (e) optionally, packing the fractions obtained in step (c) and the administration units obtained in step (d), respectively.
129. The method according to item 133, wherein in step (a) the quantity of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate exceeds the dose of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate to be contained in a single administration unit by a factor of at least 25, or at least 50, or at least 100, or at least 250, or at least 500, or at least 1000, or at least 2500, or at least 5000, or at least 10,000.
130. Particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate having a particle size distribution X90 of 500 µm or less.
131. The particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to item 130, having a particle size distribution X90 of 480 µm or less, or 460 µm or less, or 440 µm or less, or 420 µm or less, or 400 µm or less, of 380 µm or less, or 360 µm or less, or 340 µm or less, or 320 µm or less, or 300 µm or less, of 280 µm or less, or 260 µm or less, or 240 µm or less, or 220 µm or less, or 200 µm or less, of 180 µm or less, or 160 µm or less, or 140 µm or less, or 120 µm or less, or 100 µm or less, or 80 µm or less, or 60 µm or less, or 40 µm or less, or 20 µm or less.
132. The particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to any of items 130 to 131, having a particle size distribution X50 of 400 µm or less.
133. The particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to item 132, having a particle size distribution X50 of 380 µm or less, or 360 µm or less, or 340 µm or less, or 320 µm or less, or 300 µm or less, of 280 µm or less, or 260 µm or less, or 240 µm or less, or 220 µm or less, or 200 µm or less, 180 µm or less, or 160 µm or less, or 140 µm or less, or 120 µm or less, or 100 µm or less, or 80 µm or less, or 70 µm or less, or 60 µm or less, or 50 µm or less.
134. The particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to item 133, having a particle size distribution X50 of 45 µm or less, or 40 µm or less, or 35 µm or less, or 30 µm or less, or 25 µm or less, or 20 µm or less, or 15 µm or less, or 10 µm or less.
135. The particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to any of items 130 to 134, having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.
136. The particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to any of items 130 to 135, having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.
137. The particles of (i) polymorphic Form M of Compound (1), polymorphic Form H of Compound (1), polymorphic Form P of Compound (1), polymorphic Form X of Compound (1), or polymorphic Form ZA of Compound (1); or amorphous form of Compound (1); or (ii) Compound (1) • H₂O, Compound (1) • hydrate A, Compound (1) • hydrate B, Compound (1) • methanol, Compound (1) • ethanol • isopropanol, Compound (1) • acetone, Compound (1) • ethylacetate, Compound (1) • ethylacetate solvate A, Compound (1) • ethylacetate solvate B, Compound (1) • ethylacetate solvate C, Compound (1) • ethylacetate solvate D, Compound (1) • ethylacetate solvate E, Compound (1) • ethylacetate solvate F, Compound (1) • ethylacetate solvate G, Compound (1) • isopropylacetate, Compound (1) • ethylacetate • 2-methyl THF, Compound (1) • ethanol, Compound (1) • n-butylacetate, Compound (1) • n-butylacetate Solvate A, Compound (1) • n-butylacetate Solvate B, Compound (1) • n-butylacetate Solvate C, Compound (1) • heptane, Compound (1) • heptane Solvate A, Compound (1) • heptane Solvate B, Compound (1) • heptane Solvate C, Compound (1) • heptane Solvate D, Compound (1) • heptane Solvate E, Compound (1) • 2-butanone, or Compound (1) • methylacetate according to any of items 130 to 136, having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

## Claims

1. A packaging comprising a multitude
(i) of at least 2 administration units comprising a polymorphic form of Compound (1) selected from the group consisting of Form M, which is characterized as having an X-ray powder diffraction pattern obtained at room temperature using Cu K alpha radiation with the most intense characteristic peak expressed in 2-theta ± 0.2 at 19.6; Form H, which is characterized as having an X-ray powder diffraction pattern obtained at room temperature using Cu K alpha radiation with characteristic peaks expressed in 2-theta ± 0.2 at 6.6 and 17.3, wherein the peak at 6.6 is the most intense peak; Form P, which is characterized as having an X-ray powder diffraction pattern obtained at room temperature using Cu K alpha radiation with characteristic peaks expressed in 2-theta ± 0.2 at 7.0 and 15.8, wherein the peak at 7.0 is the most intense peak; Form X, which is characterized as having an X-ray powder diffraction pattern obtained at room temperature using Cu K alpha radiation with characteristic peaks expressed in 2-theta ± 0.2 at 7.5 and 12.1; and Form ZA, which is characterized as having an X-ray powder diffraction pattern obtained at room temperature using Cu K alpha radiation with characteristic peaks expressed in 2-theta ± 0.2 at 5.2 and 10.2; or an amorphous form of Compound (1), which is characterized as having a solid state C¹³ nuclear magnetic spectroscopy spectrum having peaks at 161.1, 132.9, 106.5, 43.3, 31.2, and 23.3; or
(ii) of at least 2 administration units comprising a solvate of Compound (1) selected from the group consisting of Compound (1) • H₂O, Compound (1) • methanol, Compound (1) • ethanol • isopropanol, Compound (1) • acetone, Compound (1) • ethylacetate, Compound (1) • isopropylacetate, Compound (1) • ethylacetate • 2-methyl THF, Compound (1) • ethanol, Compound (1) • n-butylacetate, Compound (1) • heptane, Compound (1) • 2-butanone, and Compound (1) • methylacetate;
wherein compound (1) has the following formula:

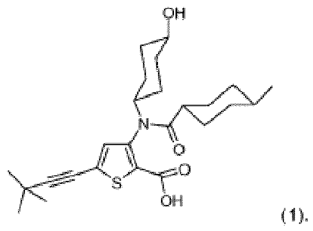

2. The packaging according to claim 1, wherein the multitude comprises at least 3, or at least 4, or at least 5, or at least 6, or at least 7, or at least 8, or at least 9, or at least 10 administration units.

3. The packaging according to any of claims 1 to 2, which comprises a material selected from the group consisting of paper, cardboard, paperboard, metal foil and plastic foil.

4. The packaging according to any of claims 1 to 3, which is a blister packaging.

5. The packaging according to any of claims 1 to 4, wherein the administration units do not substantially differ from one another in at least one property selected from the group consisting of nature of excipients; content of excipients; content of (i) Form M, Form H, Form P, Form X, or Form ZA; or content of amorphous form of Compound (1); or content of (ii) Compound (1) • H₂O, Compound (1) • methanol, Compound (1) • ethanol • isopropanol, Compound (1) • acetone, Compound (1) • ethylacetate, Compound (1) • isopropylacetate, Compound (1) • ethylacetate • 2-methyl THF, Compound (1) • ethanol, Compound (1) • n-butylacetate, Compound (1) • heptane, Compound (1) • 2-butanone, or Compound (1) • methylacetate; content of Compound (1); water content; total weight; release profile; disintegration time; size; shape; porosity; apparent density; true density; average pore size; pore size distribution; axial tensile strength; radial tensile strength; friability; and storage stability.

6. The packaging according to any of claims 1 to 5, wherein all administration units are substantially identical.

7. The packaging according to any of claims 1 to 6, wherein
Compound (1) • H₂O is selected from Compound (1) • hydrate A, which is characterized as having an X-ray powder diffraction pattern obtained at room temperature using Cu K alpha radiation with characteristic peaks expressed in 2-theta ± 0.2 at the following positions: 20.3 and 9.1; and Compound (1) • hydrate B, which is characterized as having an X-ray powder diffraction pattern obtained at room temperature using Cu K alpha radiation with characteristic peaks expressed in 2-theta ± 0.2 at the following positions: 19.0 and 9.9;
Compound (1) • methanol is characterized as having an X-ray powder diffraction pattern obtained at room temperature using Cu K alpha radiation with characteristic peaks expressed in 2-theta ± 0.2 at the following positions: 8.0 and 10.3;
Compound (1) • ethanol • isopropanol is characterized as having an X-ray powder diffraction pattern obtained at room temperature using Cu K alpha radiation with characteristic peaks expressed in 2-theta ± 0.2 at the following positions: 9.3 and 18.3;
Compound (1) • acetone is characterized as having an X-ray powder diffraction pattern obtained at room temperature using Cu K alpha radiation with characteristic peaks expressed in 2-theta ± 0.2 at the following positions: 7.7 and 16.5;
Compound (1) • ethylacetate is selected from the group consisting of Compound (1) • ethylacetate solvate A, which is characterized as having an X-ray powder diffraction pattern obtained at room temperature using Cu K alpha radiation with characteristic peaks expressed in 2-theta ± 0.2 at the following positions: 6.4, 7.3, and 9.8; Compound (1) • ethylacetate solvate B, which is characterized as having an X-ray powder diffraction pattern obtained at room temperature using Cu K alpha radiation with characteristic peaks expressed in 2-theta ± 0.2 at the following positions: 7.3 an 6.4; Compound (1) • ethylacetate solvate C, which is characterized as having an X-ray powder diffraction pattern obtained at room temperature using Cu K alpha radiation with characteristic peaks expressed in 2-theta ± 0.2 at the following positions: 12.4 and 18.8; Compound (1) • ethylacetate solvate D, which is characterized as having an X-ray powder diffraction pattern obtained at room temperature using Cu K alpha radiation with characteristic peaks expressed in 2-theta ± 0.2 at the following positions: 16.6 and 20.2; Compound (1) • ethylacetate solvate E, which is characterized as having an X-ray powder diffraction pattern obtained at room temperature using Cu K alpha radiation with characteristic peaks expressed in 2-theta ± 0.2 at the following positions: 12.4 and 18.8; Compound (1) • ethylacetate solvate F, which is characterized as having an X-ray powder diffraction pattern obtained at room temperature using Cu K alpha radiation with characteristic peaks expressed in 2-theta ± 0.2 at the following positions: 7.3, 17.1, and 22.7; and Compound (1) • ethylacetate solvate G, which is characterized as having an X-ray powder diffraction pattern obtained at room temperature using Cu K alpha radiation with characteristic peaks expressed in 2-theta ± 0.2 at the following positions: 7.5 and 12.1;
Compound (1) • isopropylacetate is characterized as having an X-ray powder diffraction pattern obtained at room temperature using Cu K alpha radiation with characteristic peaks expressed in 2-theta ± 0.2 at the following positions: 7.0 and 6.4;
Compound (1) • ethylacetate • 2-methyl THF is selected from Compound (1) • ethylacetate • 2-methyl THF solvate A, which is characterized as having an X-ray powder diffraction pattern obtained at room temperature using Cu K alpha radiation with characteristic peaks expressed in 2-theta ± 0.2 at the following positions: 5.5 and 9.3; and Compound (1) • ethylacetate • 2-methyl THF solvate B, which is characterized as having an X-raypowder diffraction pattern obtained at room temperature using Cu K alpha radiation with characteristic peaks expressed in 2-theta ± 0.2 at the following positions: 6.5 and 7.3;
Compound (1) • ethanol is characterized as having an X-ray powder diffraction pattern obtained at room temperature using Cu K alpha radiation with characteristic peaks expressed in 2-theta ± 0.2 at the following positions: 8.1 and 10.1;
Compound (1) • n-butylacetate is selected from the group consisting of Compound (1) • n-butylacetate solvate A, which is characterized as having an X-ray powder diffraction pattern obtained at room temperature using Cu K alpha radiation with characteristic peaks expressed in 2-theta ± 0.2 at the following positions: 9.7 and 16.5; Compound (1) • n-butylacetate solvate B, which is characterized as having an X-ray powder diffraction pattern obtained at room temperature using Cu K alpha radiation with characteristic peaks expressed in 2-theta ± 0.2 at the following positions: 6.4 and 6.9; and Compound (1) • n-butylacetate solvate C, which is characterized as having an X-ray powder diffraction pattern obtained at room temperature using Cu K alpha radiation with characteristic peaks expressed in 2-theta ± 0.2 at the following positions: 6.9 and 9.6;
Compound (1) • heptane is selected from the group consisting of Compound (1) • heptane solvate A, which is characterized as having an X-ray powder diffraction pattern obtained at room temperature using Cu K alpha radiation with characteristic peaks expressed in 2-theta ± 0.2 at the following positions: 10.7 and 21.3; Compound (1) • heptane solvate B, which is characterized as having an X-ray powder diffraction pattern obtained at room temperature using Cu K alpha radiation) with characteristic peaks expressed in 2-theta ± 0.2 at the following positions: 5.5 and 7.5; Compound (1) • heptane solvate C, which is characterized as having an X-ray powder diffraction pattern obtained at room temperature using Cu K alpha radiation with characteristic peaks expressed in 2-theta ± 0.2 at the following positions: 10.4 and 11.2; Compound (1) • heptane solvate D, which is characterized as having an X-ray powder diffraction pattern obtained at room temperature using Cu K alpha radiation with characteristic peaks expressed in 2-theta ± 0.2 at the following positions: 10.3 and 13.7; and Compound (1) • heptane solvate E, which is characterized as having an X-ray powder diffraction pattern obtained at room temperature using Cu K alpha radiation with characteristic peaks expressed in 2-theta ± 0.2 at the following positions: 5.0 and 10.2;
Compound (1) • 2-butanone is characterized as having a solid state C¹³ nuclear magnetic spectroscopy spectrum having peaks at: 205.7, 132.5, 127.7, 42.9, and 37.4; and
Compound (1) • methylacetateis characterized as having a solid state C¹³ nuclear magnetic spectroscopy (NMR) spectrum having peaks at: 170.5, 137.2, 106.5, 54.9, and 51.1.

8. The packaging according to any of claims 1 to 7 for use in the treatment of a disorder or a disease, wherein one or more administration units of said multitude are administered on a first day, and one or more administration units of said multitude are administered on a second day following said first day.

9. The packaging according to claim 8, wherein one or more administration units of said multitude are administered on one or more subsequent days following said second day.

10. The packaging according to any of claims 8 to 9, wherein the time interval between the administration of an administration unit and the administration of a subsequent administration unit is not more than 48 hours.

11. The packaging according to any of claims 8 to 10, wherein the disorder or disease is hepatitis C virus infection.

12. The packaging according to any of claims 8 to 11, wherein the administration units are administered orally with a liquid.

13. The packaging according to claim 12, wherein the liquid is water, milk, juice or lemonade.

14. The packaging according to any of claims 8 to 13, wherein the administered daily dose of Compound (1) is 0.001 mg to 5000 mg.

15. The packaging according to claim 14, wherein the administered daily dose of Compound (1) is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.
